# FASCICULE DE BREVET EUROPEEN

(11) **EP 0 587 499 B1**
(45) Date de publication et mention de la délivrance du brevet: **29.04.1998**
(21) Numéro de dépôt: 93402190.8
(22) Date de dépôt: 09.09.1993
(51) Int. Cl.: C07D 311/66, C07D 405/12, C07D 407/12, C07D 405/14, A61K 31/35

(54) **Composés benzopyraniques, leur procédé de préparation et leur utilisation comme protecteurs cellulaires**
Benzopyran-Verbindungen, Verfahren zu ihrer Herstellung und ihre Verwendung als Zellenschutzmittel
Benzopyran compounds, process for their preparation and their use as cell protectors

(30) Priorité: 09.09.1992 FR 9210741
(43) Date de publication de la demande: 16.03.1994
(73) Titulaire: ADIR ET COMPAGNIE, 92415 Courbevoie Cédex (FR)
(72) Inventeur: Le Baut, Guillaume, F-44230 Saint-Sebastien sur Loire (FR); Babingui, Jean-Paul, F-44000 Nantes (FR); Robert-Piessard, Sylvie, F-44200 Nantes (FR); Renard, Pierre, F-78000 Versailles (FR); Caignard, Daniel-Henri, F-75015 Paris (FR); Renaud de la Faverie, Jean-François, F-78150 Le Chesnay (FR); Adam, Gérard, F-78600 Le Mesnil le Roi (FR)

(56) Documents cités:
- WO-A-88/08424

## Description

La présente invention concerne de nouveaux composés benzopyraniques, leur procédé de préparation et les compositions pharmaceutiques qui les contiennent.

Récemment, la demande de brevet WO 88/08424 a décrit des composés de l'acide chromane-2-carboxylique et plus généralement d'acides (chroman-2-yl) alkylcarboxyliques.

Il est désormais établi que la peroxydation lipidique est un facteur pathologique majeur. Notamment, il est clair que le processus de peroxydation lipidique et les produits qu'il génère peuvent être néfastes à la viabilité cellulaire.

Les effets de la peroxydation lipidique ont été impliqués dans de nombreuses conditions pathologiques telles que l'athérosclérose, les anémies hémolytiques et dommages dus à l'ischémie-reperfusion (oxidative Damage and Repair, Chemical, Biological and Medical Aspects. 1991 Pergamon Press, page XXi). La possibilité de disposer de molécules permettant de lutter contre ce phénomène de peroxydation lipidique s'avère donc d'une grande utilité pour le clinicien pour la prévention et le traitement des pathologies impliquant un tel phénomène.

Il est également connu que les eicosanoïdes (prostaglandines et leucotriènes) issus du métabolisme de l'acide arachidonique sont à la base du mécanisme inflammatoire. Des composés, permettant l'inhibition de l'activité des enzymes lipoxygénase et/ou cycloxygénase seraient donc utiles, par exemple, dans la prévention et le traitement de l'arthrite rhumatoïde, dans l'asthme et dans l'allergie.

La demanderesse a maintenant découvert de nouveaux composés thiocarboxamides, benzopyraniques possédant une activité anti-oxydante, nettement supérieure à celles des composés qui constituent l'art antérieur le plus proche.

La demanderesse a également découvert que ces nouveaux composés permettaient une inhibition de la synthèse des eicosanoïdes ainsi que la préservation du pH intracellulaire face à une acidose. Les composés de l'invention possèdent donc une action extrêmement bénéfique en tant que protecteurs cellulaires.

Plus spécifiquement l'invention concerne de nouveaux composés benzopyraniques répondant à la formule générale (I) : 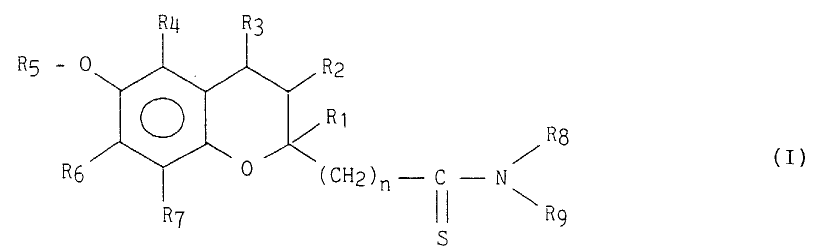 dans laquelle :
n représente un nombre entier égal à 0 ou 1,
R₁, R₂, R₃, R₄, R₆ et R₇, identiques ou différents, représentent chacun indépendamment l'un de l'autre un atome d'hydrogène ou un radical alkyl inférieur Rₐ-, où Rₐ représente un groupement alkyle linéaire ou ramifié de 1 à 8 atomes de carbone,
R₅ représente :
   - un atome d'hydrogène,
   - un groupement alkyle inférieur Rₐ-,
   - un groupement acyle inférieur Rₐ-CO-,
   - un groupement alcoxyalkyle de forme Rₐ-O-R_{b}-,
   - un groupement alcoxycarbonyle de forme Rₐ-O-CO-,
   - un groupement alcoxycarbonylalkyle de forme Rₐ-O-CO-R_{b}-,
   - un groupement carboxylalkyle de forme HOOC-Rₐ-,
      où Rₐ et R_{b}, identiques ou différents, représentent chacun indépendamment l'un de l'autre, un radical alkyle linéaire ou ramifié comportant de 1 à 8 atomes de carbone,
R₈ et R₉
   - soit forment ensemble avec l'atome d'azote qui les porte un groupement choisi parmi :
      . pipérazine,
      . pipérazine substituée,
      . pipéridine,
      . pipéridine substituée,
      . pyrrolidine,
      . pyrrolidine substituée,
      . morpholine,
      . morpholine substituée par un ou plusieurs radicaux alkyles,
      . tétrahydropyridine,
      . thiomorpholine,
      . azaspirane de 5 à 12 chaînons,
      . azaspirane de 5 à 12 chaînons substitué par un ou plusieurs radicaux alkyles ou groupement oxo,
      . azacycloalkyle mono ou bicyclique de 7 à 12 chaînons incluant éventuellement dans son squelette de 1 à 2 hétéroatomes supplémentaires choisis parmi oxygène, soufre, et azote,
      . azacycloalkyle mono ou bicyclique de 7 à 12 chaînons, substitué par un ou plusieurs radicaux alkyles ou groupements oxo, incluant éventuellement 1 à 2 hétéroatomes supplémentaires choisis parmi oxygène, soufre, et azote,
      . un groupement -NH-(CH₂)ₖ-NH₂ dans lequel k représente un nombre entier égal à 2, 3, ou 4,
      . et un groupement -NH-(CH₂)ₖ-NH₂ substitué dans lequel k est tel que défini précédemment,
étant entendu que le terme "substitué(e)" affectant ci-dessus les groupements pipérazine, pipéridine, pyrrolidine, et -NH-(CH₂)ₖ-NH₂ signifie que ces groupements peuvent être substitués par un ou plusieurs atomes d'halogène, radicaux hydroxyles, radicaux carboxyles, radicaux R₁₀, ou radicaux 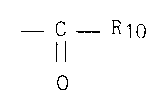 avec R₁₀ étant choisi parmi :
. alkyle,
. alkoxy,
. alcényle,
. -(CH₂)ₙ-R₁₁ ou 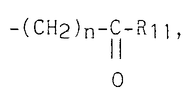 où n représente 0 ou un nombre entier de 1 à 5 et où R₁₁ représente un radical choisi parmi phényle, benzhydryle, 1,1-diphénylméthylidényle, thiényle, pyrrolyle, pyrrolidinyle, furyle, pyrimidinyle, pyridyle, benzodioxolyle, benzodioxanyle, naphtyle, quinoléinyle, isoquinoléinyle, cycloalkyle, et dicycloalkylméthyle ; le terme "cycloalkyle" représentant un groupement mono- ou bi-cyclique de 3 à 12 chaînons, ces radicaux R₁₀ pouvant eux-mêmes être substitués par un ou plusieurs radicaux choisis parmi halogène, trifluorométhyle, oxo, carboxyle, hydroxyle, alkyle, alkoxy, halogénoalkoxy, acétyle, et pyrrolidinyle,

- soit R₈ et R₉, identiques ou différents, représentent chacun indépendamment l'un de l'autre :
   . un atome d'hydrogène,
   . un groupement alkyle inférieur Rₐ- ou un groupement alkyle inférieur Rₐ- substitué,
   . un groupement alcényle inférieur ou un groupement alcényle inférieur substitué, où le groupement alcényle représente un hydrocarbure insaturé, linéaire ou ramifié, comprenant 2 à 8 atomes de carbone,
   . un groupement A-(CH₂)ₘ- ou un groupement A-(CH₂)ₘ- substitué, où m est un nombre entier égal à 0,1, ou 2 et A représente un groupement cycloalkyle comportant p atomes de carbone avec p étant un nombre entier de 3 à 7,
   . un groupement alcoxyalkyle de forme Rₐ-O-R_{b}- ou un groupement alcoxyalkyle de forme Rₐ-O-R_{b}- substitué, où Rₐ et R_{b}, identiques ou différents, représentent un radical alkyle inférieur, linéaire ou ramifié, de 1 à 8 atomes de carbone,
   . un groupement alcoxycarbonylalkyle de forme Rₐ-O-CO-R_{b}- ou un groupement alcoxycarbonylalkyle de forme Rₐ-O-CO-R_{b}- substitué, avec Rₐ et R_{b} tels que définis ci-dessus,
   . un groupement B-(CH₂)_{q}- ou un groupement B-(CH₂)_{q}- substitué, où q est un nombre entier égal à 0, 1, 2, ou 3 et B représente un radical naphtalène, 1,3-dioxane , pyrane, ou benzopyrane,
   . un groupement E-(CH₂)_{q}- ou un groupement E-(CH₂)_{q}- substitué, avec q tel que défini précédemment et E représentant un groupement azaspirane ou azacycloalkyle, non substitués ou substitués, tel que définis précédemment,
   . un groupement phényl-(CH₂)_{q}- ou un groupement phényl-(CH₂)_{q}-substitué, avec q tel que défini ci-dessus,
   . un groupement hétéroaryl-(CH₂)_{q}- ou un groupement hétéroaryl-(CH₂)_{q}- substitué, avec q tel que défini ci-dessus et où l'hétéroaryle est choisi parmi :
      furane, quinoléine, isoquinoléine, pyridine, thiophène, thiazole, isothiazole, oxazole, isoxazole, naphtyridine, benzofurane, β-carboline, ou Υ-carboline,
   . un groupement guanidino ou amidino, non substitué ou substitué par un ou plusieurs alkyles linéaires ou ramifiés de 1 à 6 atomes de carbone,
   . ou un des radicaux D₁ à D₄ suivants : 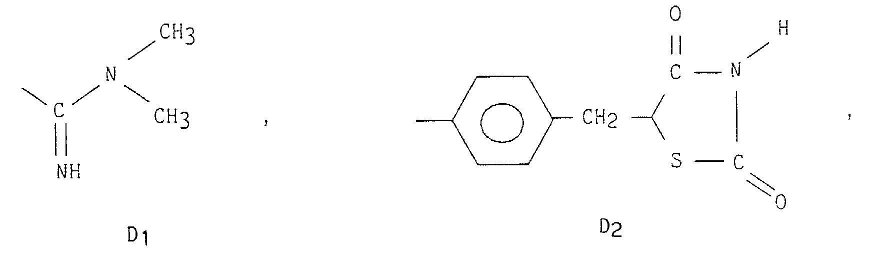 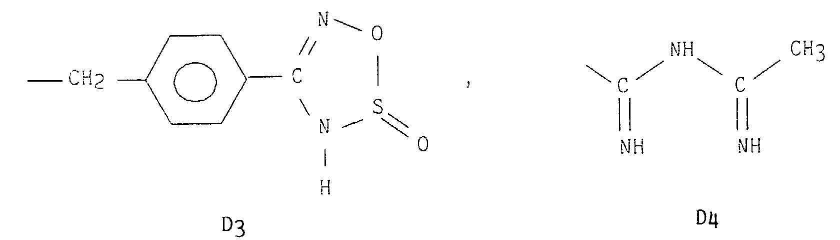
étant entendu que
lors de cette description de la formule générale (I), le terme "substitué" affectant les groupements tels que définis précédemment : alkyle inférieur Ra-, alcényle inférieur, A-(CH₂)ₘ-, alcoxyalkyle Rₐ-O-R_{b}-, alcoxycarbonylalkyle Rₐ-O-CO-R_{b}-, B-(CH₂)_{q}-, phényl-(CH₂)_{q}-, hétéroaryl-(CH₂)_{q}-, signifie, lorsque cela n'est pas précisé, que ces groupements peuvent être substitués par un ou plusieurs radicaux, identiques ou différents, et représentant chacun indépendamment l'un de l'autre :
un groupement alkyle inférieur R_{c}-,
alcoxy inférieur R_{c}-O-,
acyle inférieur R_{c}-CO-,
trifluorométhyle,
carboxyle,
hydroxyle,
oxo,
guanidino,
amidino,
ou un atome d'halogène,
où R_{c} représente un groupement alkyle linéaire ou ramifié de 1 à 6 atomes de carbone,
leurs isomères optiques, sous forme pure ou sous forme de mélange,
ainsi que, le cas échéant, leurs sels d'addition à un acide ou à une base pharmaceutiquement acceptable.

Parmi les acides ou les bases pharmaceutiquement acceptables que l'on peut utiliser pour salifier les composés de l'invention, on peut citer, à titre d'exemples et de façon non limitative, les acides chlorydrique, bromhydrique, sulfurique, nitrique, oxalique, malique, maléique, succinique, tartrique, méthanesulfonique, camphorique, camphosulfonique, la soude, la potasse, la triéthylamine, la diéthylamine, l'éthanolamine, l'arginine, la lysine, et la diéthanolamine.

L'invention s'étend au procédé d'obtention des composés de formule (I), caractérisé en ce que l'on utilise comme matière première un composé de formule (II) : 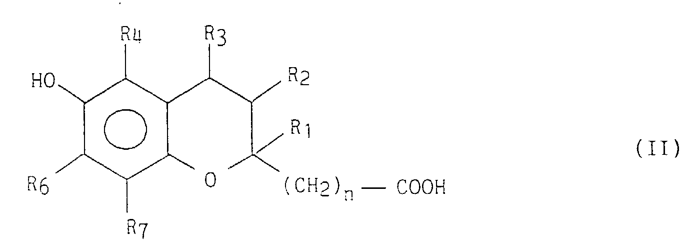 avec R₁, R₂, R₃, R₄, R₆, R₇ et n ayant la même définition que dans la formule générale (I),
que l'on peut estérifier, en milieu basique anhydre, par un composé R₅''-Hal ou R₅''-O-R₅'', où Hal représente un atome d'halogène et où R₅'' représente un groupement acyle inférieur Rₐ-CO- dans lequel Ra est tel que défini dans la formule (I),
pour obtenir un composé de formule (III) : 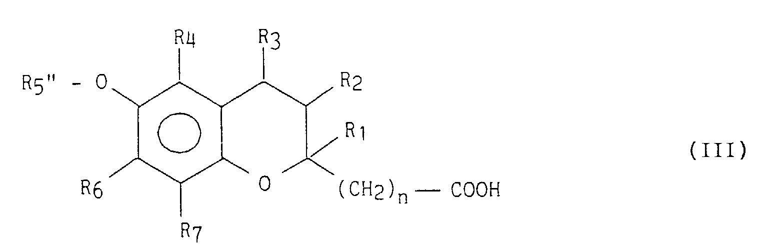 avec R₁, R₂, R₃, R₄, R₆, R₇, n, et R₅'' tels que définis précédemment, qui est transformé en son halogénure par action d'un agent d'halogénation, puis traité, dans un solvant approprié, en présence d'un agent alcalin, par une amine de formule (IV) : 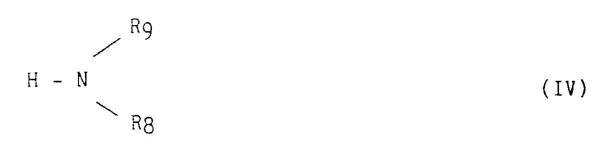 avec R₈ et R₉ ayant la même signification que dans la formule (I) pour obtenir un composé de formule (Iₐ) : 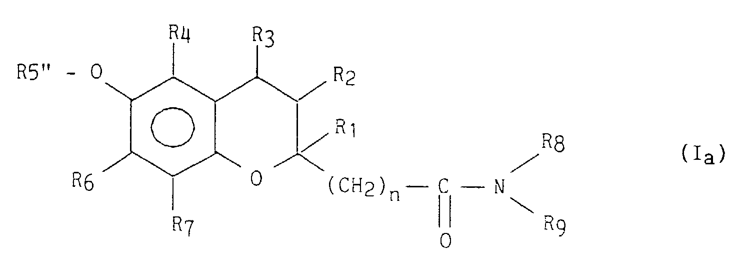 avec R₁, R₂, R₃, R₄, R₆, R₇, R₈, R₉, n, et R₅'' tels que définis précédemment,
que l'on peut ensuite,
- saponifier par action d'un hydroxyde de métal alcalin ou alcalino-terreux en un composé de formule (I_{b}) : 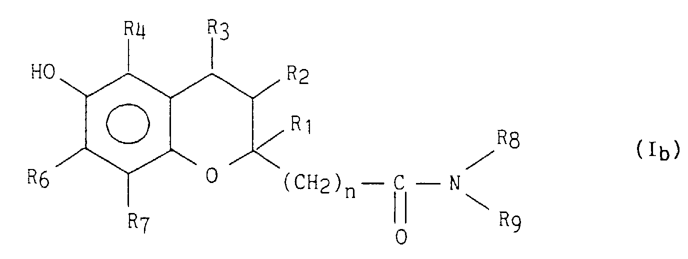 avec R₁, R₂, R₃, R₄, R₆, R₇, R₈, R₉ et n tels que définis précédemment,
- puis, le cas échéant, éthérifié par action d'un dérivé de formule R₅'''-O-R₅''' ou R₅'''-Hal', où Hal' représente un atome d'halogène et R₅''' représente un groupement alkyle inférieur Rₐ, un groupement acyle inférieur Ra-CO-, un groupement alcoxyalkyle de forme Rₐ-O-R_{b}-, un groupement alcoxycarbonyle de forme Ra-O-CO- un groupement alcoxycarbonylalkyle de forme Rₐ-O-CO-R_{b}-, ou un groupement carboxylalkyle de forme HOOC-Rₐ-, avec Rₐ et R_{b} tels que définis dans la formule (I), pour obtenir un composé de formule (I_{c}) : 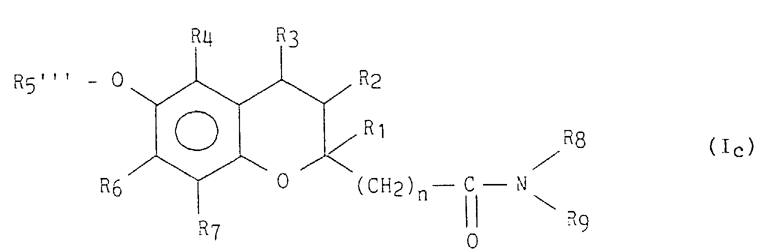 avec R₁, R₂, R₃, R₄, R₆, R₇, R₈, R₉, n, et R₅''' tels que définis précédemment,
composés de formule (Ia), (Ib), et (Ic) qui sont ensuite soumis à l'action du réactif de Lawesson pour obtenir les composés de formule (I) correspondants,

les composés de formule (I) pouvant être, si on le désire :
- purifiés,
- séparés en leurs isomères optiques, sous forme pure ou sous forme de mélange,
- ou transformés en leurs sels d'addition à une base ou à un acide pharmaceutiquement acceptable.

L'invention s'étend également au procédé d'obtention des composés de formule (I') : 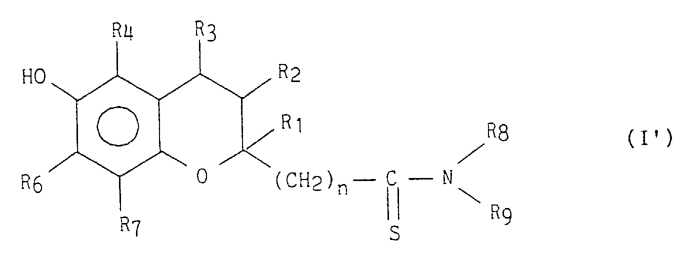 dans laquelle R₁, R₂, R₃, R₄, R₆, R₇, R₈, R₉ et n sont tels que définis dans la formule (I),
cas particulier des composés de formule (I) dans lesquels R₅ représente un atome d'hydrogène,
et des composés de formule (I'a) de formule : 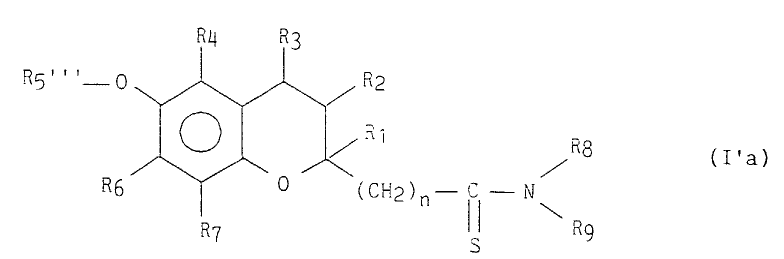 dans laquelle R₁, R₂, R₃, R₄, R₆, R₇, R₈, R₉, n sont tel que définis dans la formule (I) et R₅''' est tel que défini précédemment,
cas particulier des composés de formule (I) dans lesquels R₅ représente un radical R₅''',
caractérisé en ce que l'on saponifie, par action d'un hydroxyde de métal alcalin ou alcalino-terreux, un composé de formule (I'') : 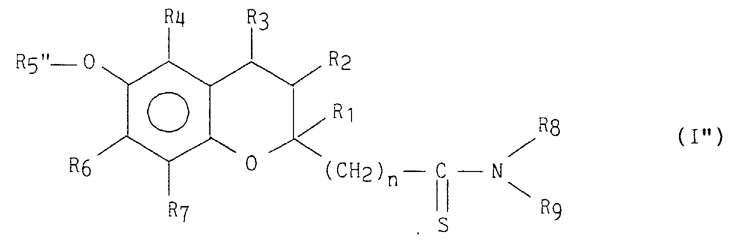 dans laquelle R₁, R₂, R₃, R₄, R₆, R₇, R₈, R₉, et n sont tels que définis précédemment et R₅'' représente un groupement acyle inférieur Ra-CO- dans lequel Ra est tel que défini dans la formule (I), cas particulier des composés de formule (I) dans lesquels R₅ représente un radical R₅'', pour obtenir le composé de formule (I') correspondant, que l'on éthérifie ou estérifie, le cas échéant, par action d'un composé de formule R₅'''-O-R₅''' ou R₅'''-Hal'', où Hal" représente un atome d'halogène et R₅''' est tel que défini précédemment, pour obtenir le composé de formule (I'a) correspondant,
composés de formule (I') et (I'a) qui peuvent être, si on le désire :
- purifiés,
- séparés en leurs isomères optiques, sous forme pure ou sous forme de mélange,
- ou transformés en leurs sels d'addition à une base ou à un acide pharmaceutiquement acceptable.

Comparés aux composés de l'art antérieur, les composés de la présente invention possèdent de façon surprenante des propriétés antioxydantes très importantes. Les études pharmacologiques ont notamment montré que ces composés étaient doués d'activités protectrices remarquables dans le cadre des processus de peroxydations des lipides cellulaires et des lipoprotéines de faible densité (LDL). Ces activités sont pour certains des composés de l'invention 100 fois supérieures à celle du composé le plus proche de l'art antérieur, c'est à dire l'exemple 102 de la demande WO 88/08424. (Exemples pharmacologiques B et C de la présente demande).

Par ailleurs, certains composés de la présente invention présentent la particularité d'avoir un puissant effet inhibiteur de la biosynthèse des eicosanoïdes, lesquels proviennent de composés peroxidés, générateurs potentiels de radicaux libres, effet inhibiteur que ne possède pas le composé le plus proche de l'art antérieur.

En outre, la demanderesse a découvert que les composés de l'invention étaient d'excellents protecteurs du pH intracellulaire face à une acidification intracellulaire, une des causes principales de l'ischémie tissulaire. En effet, les composés de l'invention se sont révélés être de puissants inhibiteurs des transporteurs de bicarbonate et notamment de l'échangeur Cl-/HCO₃- sodium-indépendant des cellules cardiaques (cardiocytes) en culture. (Exemple pharmacologique D de la présente demande). La fuite de bicarbonate hors de la cellule est ainsi stoppée et permet la neutralisation d'une acidification cellulaire et des traumatismes ioniques et métaboliques qui lui sont associés.

Les composés de la présente invention peuvent ainsi être utilisés dans le traitement ou la prévention des désordres ischémiques centraux ou périphériques, des maladies inflammatoires, de l'arthrite rhumatoïde, des maladies métaboliques, de l'athérome, de l'artériosclérose, des maladies respiratoires, de l'asthme, de l'emphysème, des maladies d'origine immunologique, du lupus érythémateux, des réactions allergiques, du vieillissement cérébral ou cutané ainsi que dans la prévention et le traitement des dommages dus aux traumatismes chirurgicaux tels que la reperfusion d'organes.

La présente invention a également pour objet les compositions pharmaceutiques contenant un composé de formule (I), ou un de ses sels d'addition à un acide ou une base pharmaceutiquement acceptable, en combinaison avec un ou plusieurs excipients pharmacologiquement acceptables.

Parmi les compositions pharmaceutiques selon l'invention, on pourra citer plus particulièrement celles qui conviennent à l'administration orale, percutanée, cutanée, parentérale, nasale, rectale, perlinguale, oculaire ou pulmonaire et notamment les préparations injectables ou buvables, les aérosols, les gouttes oculaires ou nasales, les comprimés simples, pelliculés ou dragéifiés, les gélules, les capsules, les crèmes, pommades, gels dermiques, les pilules, les paquets, les sachets, les granulés et les suppositoires.

La posologie varie selon l'âge, le poids et le sexe du patient, la voie d'administration, la nature et l'intensité de l'affection, et selon les éventuels traitements associés. Les doses s'échelonnent entre 0,5 mg à 2 g par jour, particulièrement entre 0,5 mg à 100 mg par jour, par exemple entre 10 mg à 100 mg par jour.

Les exemples qui suivent illustrent l'invention mais ne la limitent en aucune façon.

Les matières premières sont décrites dans la littérature ou sont facilement accessibles à l'homme de l'art.

Les spectres infra rouge sont réalisés en pastille de bromure de potassium renfermant environ 1 % du produit à analyser.

### Exemple 1 : N-phényl (6-acétoxy-3,4-dihydro-2,5,7,8-tétraméthyl-2H[1]-benzopyran-2-yl) thiocarboxamide

### STADE A : Acide (6-acétoxy-3,4-dihydro-2,5,7,8-tétraméthyl-2H[1]-benzopyran-2-yl) carboxylique

Dissoudre 50 grammes (0,2 mole) d'acide (6-hydroxy-3,4-dihydro-2,5,7,8-tétraméthyl-2H[1]-benzopyran-2-yl) carboxylique (ou acide (6-hydroxy-3,4-dihydro-2,5,7,8-tétraméthyl-2H-1-benzopyran-2-yl)carboxylique) dans 150 cm³ de pyridine anhydre. Ajouter goutte à goutte 9,4 cm³ (0,1 mole) d'anhydride acétique sous courant d'azote. Agiter pendant 2 h à une température de 30°C. Après refroidissement, verser le mélange sur de la glace, extraire le produit attendu par de l'éther éthylique, laver la phase organique par une solution d'acide chlorhydrique 0,2N , puis à l'eau jusqu'à neutralité. Après évaporation du solvant, on recueille une masse huileuse qui cristallise après trituration dans l'éther diisopropylique.

### STADE B : N-phényl (6-acétoxy-3,4-dihydro-2,5,7,8-tétraméthyl-2H[1]-benzopyran-2-yl) carboxamide

Dans un ballon, introduire 3,25 g (11,1 mmoles) du composé obtenu au stade précédent et 40 cm³ de benzène anhydre. Après dissolution, ajouter 1,2 cm³ de chlorure de thionyle.
Chauffer au reflux pendant 3 h. Laisser refroidir. Chasser le solvant sous vide. Reprendre le résidu par 30 cm³ de benzène anhydre, évaporer à nouveau le solvant de façon à éliminer l'excès de chlorure de thionyle. Dissoudre le chlorure d'acide ainsi obtenu dans 20 cm³ de dichloroéthane. D'autre part, mettre en solution 1,04 g (11,1 mmoles) d'aniline et 4,7 cm³ de triéthylamine dans 20 cm³ de dichloroéthane. Sur ce mélange, verser goutte à goutte la solution du chlorure d'acide. Laisser agiter à la température ordinaire pendant 2 h. Chasser le solvant sous vide. Reprendre le résidu par 30 cm³ d'eau, neutraliser avec une solution saturée de bicarbonate de sodium. Extraire le produit par du dichlorométhane. Laver la phase organique à l'eau, puis sécher sur sulfate de sodium anhydre. Après élimination du solvant, purifier le produit par passage sur colonne de gel de silice en éluant avec de l'éther isopropylique. Masse du produit obtenu : 3,3 g.
Rendement : 80,9 %
- Point de fusion : 104 - 105°C
- Caractéristiques spectrales en infra rouge :

| | |
|---|---|
| νC=O | 1750 cm⁻¹ |
| νC=O (amide) | 1685 cm⁻¹ |
| νC=C, | 1595 cm⁻¹ |
| νSNH | 1520 cm⁻¹ |

### STADE C : N-phényl (6-acétoxy-3,4-dihydro-2,5,7,8-tétraméthyl-2H[1]-benzopyran-2-yl) thiocarboxamide

Dissoudre 5,54 g (17 mmoles) du N-phényl (6-acécoxy-3,4-dihydro-2,5,7,8-tétraméthyl-2H[1]-benzopyran-2-yl) carboxamide obtenu au stade précédent dans 125 cm³ de toluène anhydre. Ajouter 4,15 g (10,2 mmoles) de réactif de lawesson, puis chauffer au reflux pendant 6 heures. Evaporer le solvant et purifier le composé du titre sur une colonne de gel de silice en éluant avec du dichlorométhane. On obtient une poudre cristalline jaune que l'on cristallise dans l'éther diisopropylique.
Rendement 90 %

### Exemple 2 : N-phényl (6-hydroxy-3,4-dihydro-2,5,7,8-tétraméthyl-2H[1]-benzopyran-2-yl) thiocarboxamide

Dissoudre dans un ballon à deux cols, 2,75 g (7,48 mmoles) du composé obtenu au stade B de l'exemple 1 dans 60 cm³ d'éthanol à 80 %. Ajouter sous courant d'azote, 18 cm³ d'hydroxyde de sodium 2,5N. Laisser agiter à la température ordinaire pendant 2 h. Diluer le mélange à l'eau, acidifier avec de l'acide acétique, puis extraire le produit avec du dichlorométhane. Laver la phase organique à l'eau puis sécher. Purifier par passage sur colonne de gel de silice en éluant avec de l'éther isopropylique pour obtenir le N-phényl (6-hydroxy-3,4-dihydro-2,5,7,8-tétraméthyl-2H[1]-benzopyran-2-yl) carboxamide (rendement : 86 % ; point de fusion : 107 - 109°C) puis opérer comme dans le stade C de l'exemple 1 en remplaçant le N-phényl (6-acétoxy-3,4-dihydro-2,5,7,8-tétraméthyl-2H[1]-benzopyran-2-yl) carboxamide par le N-phényl (6-hydroxy-3,4-dihydro-2,5,7,8-tétraméthyl-2H[1]-benzopyran-2-yl) carboxamide.
Rendement : 34 %
- Point de fusion : 150 - 152°C (éther diisopropylique)
- Caractéristiques spectrales en infra rouge :

| | |
|---|---|
| νOH | 3500 cm⁻¹ |
| νNH | 3320 cm⁻¹ |
| νC=S, | 720 cm⁻¹ |

### 2ème procédé :

Dissoudre 5,74 mmoles du composé de l'exemple 1 dans 80 cm³ d'éthanol à 60 %, ajouter sous azote 14 cm³ d'hydroxyde de sodium 2,5N. Agiter pendant 3 h puis diluer le mélange à l'eau et acidifier avec de l'acide acétique. Extraire le produit avec du dichlorométhane, laver la phase organique à l'eau, sécher sur sulfate de sodium anhydre puis évaporer le solvant. Reprendre l'huile obtenue par 15 cm³ d'éther isopropylique et isoler le produit en utilisant les techniques classiques de séparation chromatographique ou cristallographique.

On obtient le produit du titre :
Rendement : 89 %
- Point de Fusion : 150 - 152°C
- Solvant : éther diisopropylique

### Exemple 3 : N-(2,4,5-triméthylphényl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 2,4,5-triméthylaniline, on obtient le produit du titre.

### Exemple 4 : N-(2,4,5-triméthylphényl) (6-hydroxy-3,4-dihydro-2,5,7,8-tétraméthyl-2H[1]-benzopyran-2-yl) thiocarboxamide

En opérant comme dans l'exemple 2, mais en utilisant le N-(2,4,5-triméthylphényl) (6-acétoxy-3,4-dihydro-2,5,7,8-tétraméthyl-2H[1]-benzopyran-2-yl) carboxamide (point de fusion : 160-162°C), on obtient le composé du titre :
- Point de fusion : 124 - 125°C (éther diisopropylique)
- Caractéristiques spectrales en infra-rouge :

| | |
|---|---|
| νNH | 3320 cm⁻¹ |
| νOH | 3450 cm⁻¹ |
| νC=S | 1165 cm⁻¹ |

### Exemple 5 : N-(4,6-diméthylpyridin-2-yl) (6-acétoxy-3,4-dihydro-2,5,7,8-tétraméthyl-2H[1]-benzopyran-2-yl) thiocarboxamide

En remplaçant dans le stade 3 de l'exemple 1 l'aniline par la (4,6-diméthylpyridin-2-yl)amine, on obtient le produit du titre.

### Exemple 6 : N-(4,6-diméthylpyridin-2-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 5, on obtient le produit du titre
- Rendement : 60 %
- Point de fusion : 180-190°C (éther diisopropylique)
- Caractéristiques spectrales en infra-rouge :

| | |
|---|---|
| ν(OH) | 3420 cm⁻¹ |
| ν(NH) | 3310 cm⁻¹ |
| ν(C=C, N=C) | 1620, 1570 cm⁻¹ |

### Exemple 7 : N-(3,4,5-triméthoxyphényl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 3,4,5-triméthoxyaniline, on obtient le produit du titre.

### Exemple 8 : N-(3,4,5-triméthoxyphényl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 7, on obtient le produit du titre :
- Rendement : 82 %
- Point de fusion : 147 - 148°C (éther diisopropylique)
- Caractéristiques spectrales en infra-rouge :

| | |
|---|---|
| νOH | 3460 cm⁻¹ |
| νNH | 3320 cm⁻¹ |

### Exemple 9 : N-hexyl N-(4,6-diméthylpyridin-2-yl) (6-acétoxy-3,4-dihydro-2,5,7,8-tétraméthyl-2H[1]-benzopyran-2-yl) thiocarboxamide

En remplaçant dans le stade 3 de l'exemple 1 l'aniline par la N-(4,6-diméthylpyridin-2-yl) hexylamine, on obtient le produit du titre.

### Exemple 10 : N-hexyl N-(4,6-diméthylpyridin-2-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 9, on obtient le produit du titre.

### Exemple 11 : N-phényl N-(butèn-3 yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la N-(butèn-3 yl) aniline, on obtient le produit du titre.

### Exemple 12 : N-phényl N-(butèn-3 yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 11, on obtient le produit du titre.

### Exemple 13 : N-furfuryl (6-acétoxy-3,4-dihydro-2,5,7,8-tétraméthyl-2H[1]-benzopyran-2-yl) thiocarboxamide

En remplaçant dans le stade B de l'exemple 1 l'aniline par la furfurylamine, on obtient le produit du titre.

### Exemple 14 : N-furfuryl (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 13, on obtient le produit du titre.

### Exemple 15 : N-(4-hydroxy-2,3-diméthylphényl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 4-hydroxy-2,3-diméthylaniline, on obtient le produit du titre.

### Exemple 16 : N-(4-hydroxy-2,3-diméthylphényl) (6-hydroxy-3,4-dihydro-2,5,7,8-tétraméthyl-2H[1]-benzopyran-2-yl) thiocarboxamide

En opérant comme dans l'exemple 2 (2ème procédé). mais en remplaçant le composé de l'exemple 1 par le composé de l'exemple 15, on obtient le produit du titre.

### Exemple 17 : N-(5,7-diméthylnaphtyridin-2-yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 2-amino-5,7-diméthylnaphtyridine, on obtient le produit du titre.

### Exemple 18 : N-(5,7-diméthylnaphtyridin-2-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 17, on obtient le produit du titre.

### Exemple 19 : N-cyclopropylméthyl N-(4,6-diméthylpyridin-2-yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la N-(4,6-diméthylpyridin-2-yl) cyclopropylméthylamine et en changeant la durée de l'étape de chauffage au reflux de 3 heures en 24 heures, on obtient le produit du titre.

### Exemple 20 : N-cyclopropylméthyl N-(4,6-diméthylpyridin-2-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 19, on obtient le produit du titre.

### Exemple 21 : N-(4-méthylquinoléin-2-yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 2-amino-4-méthylquinoléine, on obtient le produit du titre.

### Exemple 22 : N-(4-méthylquinoléin-2-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 21, on obtient le produit du titre.

### Exemple 23 : N-isobutyl N-(4,6-diméthylpyridin-2-yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la N-(4,6-diméthylpyridin-2-yl) isobutylamine, on obtient le produit du titre.

### Exemple 24 : N-isobutyl N-(4,6-diméthylpyridin-2-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 23, on obtient le produit du titre.

### Exemple 25 N-(2,6-diméthylphényl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 2,6-diméthylaniline, on obtient le produit du titre.

### Exemple 26 : N-(2,6-diméthylphényl) (6-hydroxy-3,4-dihydro-2,5,7,8-tétraméthyl-2H[1]-benzopyran-2-yl) thiocarboxamide

En opérant comme dans l'exemple 2, mais en utilisant le N-(2,6-diméthylphényl) (6-acétoxy-3,4-dihydro-2,5,7,8-tétraméthyl-2H[1]-benzopyran-2-yl) carboxamide, on obtient le produit du titre.
- Point de fusion : 122 - 123°C (éther diisopropylique)
- Caractéristiques spectrales en infra rouge :

| | |
|---|---|
| νOH | 3400 cm⁻¹ |
| νNH | 3320 cm⁻¹ |
| νC=S | 1040 cm⁻¹ |

### Exemple 27 : N-(2-carboxy-4,5-diméthoxyphényl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 2-carboxy-4,5-diméthoxy-aniline, on obtient le produit du titre.

### Exemple 28 : N-(2-carboxy-4,5-diméthoxyphényl) (6-hydroxy-3,4-dihydro 2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 27, on obtient le produit du titre.

### Exemple 29 : N-(3,5-dichloro-4-hydroxyphényl) (6-acétoxy-3,4-dihydro 2,5,7,8-tétraméthyl-2H[1]-benzopyran-2-yl) thiocarboxamide

En remplaçant dans le stade B de l'exemple 1 l'aniline par la 3,5-dichloro-4-hydroxyaniline, on obtient le produit du titre.

### Exemple 30 : N-(3,5-dichloro-4-hydroxyphényl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 29 on obtient le produit du titre.

### Exemple 31 N-(2-carboxy-4,6-diméthylphényl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 2-carboxy-4,5-diméthylaniline, on obtient le produit du titre.

### Exemple 32 : N-(2-carboxy-4,6-diméthylphényl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 31, on obtient le produit du titre.

### Exemple 33 : N-(2,4,6-triméthylphényl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 2,4,6-triméthylaniline, on obtient le produit du titre.

### Exemple 34 N-(2,4,6-triméthylphényl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 33, on obtient le produit du titre.

### Exemple 35 : N-(2-méthylquinoléin-4-yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 4-amino-2-méthylquinoléine, on obtient le produit du titre.

### Exemple 36 : N-(2-méthylquinoléin-4-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé ce l'exemple 35, on obtient le produit du titre.

### Exemple 37 : 1-oxa-2-oxo-3,8-diaza-8-((6-acétoxy-3,4-dihydro-2,5,7,8-tétraméthyl-2H[1]-benzopyran-2-yl)thiocarbonyl ) spiro[4,5]décane

En remplaçant au stade B de l'exemple 1 l'aniline par le 1-oxa-2-oxo-3,8-diaza-spiro[4,5]décane, on obtient le produit du titre.

### Exemple 38 : 1-oxa-2-oxo-3,8-diaza-8-((6-hydroxy-3,4-dihydro-2,5,7,8-tétraméthyl-2H[1]-benzopyran-2-yl)thiocarbonyl) spiro[4,5]décane

En opérant comme dans l'exemple 2 (2ème procédé), mais en remplaçant le composé de l'exemple 1 par le composé de l'exemple 37, on obtient le produit du titre.

### Exemple 39 : N-(4-chloronapht-1-yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par le 1-amino-4-chloronaphtalène, on obtient le produit du titre.

### Exemple 40 : N-(4-chloronapht-1-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple i par le composé de l'exemple 39, on obtient le produit du titre.

### Exemple 41 : N-(napht-2-yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par le 2-aminonaphtalène, on obtient le produit du titre.

### Exemple 42 : N-(napht-2-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 41, on obtient le produit du titre.

### Exemple 43 : N-(isoquinoléin-5-yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 5-amino-isoquinoléine, on obtient le produit du titre.

### Exemple 44 : N-(isoquinoléin-5-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 43, on obtient e produit du titre.

### Exemple 45 N-(thiazol-2-yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par le 2-aminothiazole, on obtient le produit du titre.

### Exemple 46 : N-(thiazol-2-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 45, on obtient le produit du titre.

### Exemple 47 : N-{4-[(2,4-dioxo-5-thiazolidinyl)méthyl]phényl} (6-acétoxy-3,4-dihydro-2,5,7,8-tétraméthyl-2H[1]-benzopyran-2-yl) thiocarboxamide

En remplaçant dans le stade B de l'exemple 1 l'aniline par la N-{4-[(2,4-dioxo-5-thiazolidinyl)méthyl]phényl}amine, on obtient le produit du titre.

### Exemple 48 : N-{4-[(2,4-dioxo-5-thiazolidinyl)méthyl]phényl} (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 47, on obtient le produit du titre.

### Exemple 49 : N-((thién-2-yl)méthyl) (6-acétoxy-3,4-dihydro-2,5,7,8-tétraméthyl-2H[1]-benzopyran-2-yl) thiocarboxamide

En remplaçant dans le stade B de l'exemple 1 l'aniline par la 2-thiophèneméthylamine, on obtient le produit du titre.

### Exemple 50 : N-((thién-2-yl)méthyl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 49, on obtient le produit du titre.

### Exemple 51 : N-butyl N-phényl (6-acétoxy-3,4-dihydro-2,5,7,8-tétraméthyl-2H[1]-benzopyran-2-yl) thiocarboxamide

En remplaçant dans le stade B de l'exemple 1 l'aniline par la N-butylaniline, on obtient le produit du titre.

### Exemple 52 : N-butyl N-phényl (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 51, on obtient le produit du titre.

### Exemples 53 à 62 :

De même, en suivant les exemples 1 et 2, et en utilisant les amines appropriées, on obtient les produits suivants :

### Exemple 53 : N-(méthoxyéthyl) (6-acétoxy-3,4-dihydro-2,5,7,8-tétraméthyl-2H[1]-benzopyran-2-yl) thiocarboxamide

### Exemple 54 N-(méthoxyéthyl) (6-hydroxy-3,4-dihydro-2,5,7,8-tétraméthyl-2H[1]-benzopyran-2-yl) thiocarboxamide

### Exemple 55 : N-phényl N-éthoxycarbonylméthyl (6-acétoxy-3,4-dihydro-2,5,7,8-tétraméthyl-2H[1]-benzopyran-2-yl) thiocarboxamide

### Exemple 56 : N-phényl N-éthoxycarbonylméthyl (6-hydroxy-3,4-dihydro-2,5,7,8-tétraméthyl-2H[1]-benzopyran-2-yl) thiocarboxamide

### Exemple 57 : N-guanidino (6-acétoxy-3,4-dihydro-2,5,7,8-tétraméthyl-2H[1]-benzopyran-2-yl) thiocarboxamide

### Exemple 58 : N-guanidino (6-hydroxy-3,4-dihydro-2,5,7,8-tétraméthyl-2H[1]-benzopyran-2-yl) thiocarboxamide

### Exemple 59 : 1-((6-acétoxy-3,4-dihydro-2,5,7,8-tétraméthyl-2H[1]-benzopyran-2-yl) thiocarbonyl) 3,3-diméthylguanidine

### Exemple 60 : 1-((6-hydroxy-3,4-dihydro-2,5,7,8-tétraméthyl-2H[1]-benzopyran-2-yl) thiocarbonyl) 3,3-diméthylguanidine

### Exemple 61 : N-{4-[(2-oxo-1,2,3,5-oxathiadiazol-4-yl)méthyl]phényl} (6-acétoxy-3,4-dihydro-2,5,7,8-tétraméthyl-2H[1]-benzopyran-2-yl) thiocarboxamide

### Exemple 62 : N-{4-[(2-oxo-1,2,3,5-oxathiadiazol-4-yl)méthyl]phényl) (6-hydroxy-3,4-dihydro-2,5,7,8-tétraméthyl-2H[1]-benzopyran-2-yl) thiocarboxamide

### Exemple 63 : N-(1,3-dihydroxy-2-méthylprop-2-yl) (6-acétoxy-3,4-dihydro-2,5,7,8-tétraméthyl-2H[1]-benzopyran-2-yl) thiocarboxamide

En remplaçant dans le stade B de l'exemple 1 l'aniline par le 2-amino-2-méthyl-1,3-propanediol, on obtient le produit du titre.

### Exemple 64 : N-(2,2,5-triméthyl-1,3-dioxan-5-yl) (6-acétoxy-3,4-dihydro 2,5,7,8-tétraméthyl-2H[1]-benzopyran-2-yl) thiocarboxamide

Dissoudre 3 grammes (7,9 mmoles) du composé de l'exemple 63 dans 45 cm³ de 2,2-diméthoxypropane, ajouter 15 cm³ de diméthylformamide anhydre puis 60 milligrammes d'acide paratoluène sulfonique. Chauffer au reflux pendant une heure. Evaporer l'excès de 2,2-diméthoxypropane sous vide. Diluer le mélange résiduel à l'eau. Extraire le produit avec du dichlorométhane. Laver la phase organique avec une solution de bicarbonate de sodium puis à l'eau. Purifier le composé du titre par passage sur colonne de gel de silice en éluant avec de l'éther éthylique. On obtient le produit du titre.

### Exemple 65 : N-(2,2,5-triméthyl-1,3-dioxan-5-yl) (6-hydroxy-3,4-dihydro-2,5,7,8-tétraméthyl-2H[1]-benzopyran-2-yl) thiocarboxamide

En opérant comme dans l'exemple 2 (2ème procédé), mais en remplaçant le composé de l'exemple 1 par le composé de l'exemple 64, on obtient le produit du titre.

### Exemple 66 : N-(4,6-diméthylpyridin-2-yl) (6-méthoxyméthoxy-3,4-dihydro-2,5,7,8-tétraméthyl-2H[1]-benzopyran-2-yl) thiocarboxamide

### STADE A : Acide (6-méthoxyméthoxy-3,4-dihydro-2,5,7,8-tétraméthyl-2H[1]-benzopyran-2-yl) carboxylique

Dissoudre 50 grammes (0,2 mole) d'acide (6-hydroxy-3,4-dihydro-2,5,7,8-tétraméthyl-2H[1]-benzopyran-2-yl) carboxylique dans 150 cm³ de pyridine anhydre. Ajouter goutte à goutte 16,34 cm³ (0,2 mole) de bromure d'oxyde de méthyle. Agiter pendant 2 heures puis verser la solution sur de la glace. Acidifier avec de l'acide acétique, puis extraire avec du chlorure de méthylène. Laver la phase organique à l'eau et sécher sur sulfate de sodium anhydre, puis purifier le produit du titre par passage sur colonne de gel de silice en éluant avec du chlorure de méthylène.

### STADE B : N-(4,6-diméthylpyridin-2-yl) (6-méthoxyméthoxy-3,4-dihydro 2,5,7,8-tétraméthyl-2H[1]-benzopyran-2-yl) carboxamide

Dissoudre 5,4 grammes (18,47 mmoles) du composé au stade précédent dans 30 cm³ de benzène anhydre, ajouter 2,2 cm³ (27,41 mmoles) de chlorure de thionyle, chauffer au reflux pendant 3 heures, chasser le solvant sous vide en éliminant l'excès de chlorure de thionyle. Dissoudre le chlorure d'acide ainsi obtenu dans 30 cm³ de dichloroéthane.
Dans un autre récipient, dissoudre 2,26 grammes (18,5 mmoles) de 2-amino-4,6-diméthylpyridine dans 20 cm³ de dichloroéthane, ajouter 7,7 cm³ de triéthylamine et verser goutte à goutte dans ce mélange la solution de chlorure d'acide précédente. Après 8 heures d'agitation, évaporer le solvant sous vide, reprendre le résidu par 30 cm³ d'eau, neutraliser avec une solution de NaHCO₃, extraire par du chlorure de méthylène, laver la phase organique à l'eau, puis la sécher sur sulfate de sodium. Après évaporation du solvant, purifier par chromatographie sur gel de silice en éluant avec du chlorure de méthylène.

### STADE C : N-(4,6-diméthylpyridin-2-yl) (6-méthoxyméthoxy-3,4-dihydro 2,5,7,8-tétraméthyl-2H[1]-benzopyran-2-yl) thiocarboxamide

En procédant comme dant le stade C de l'exemple 1 mais en remplaçant le N-phényl (6-acétoxy-3,4-dihydro 2,5,7,8-tétraméthyl-2H[1]-benzopyran-2-yl) thiocarboxamide par le composé obtenu au stade B précédent, on obtient le composé du titre.

### Exemple 67 : N-(4,6-diméthylpyridin-2-yl) (6-éthoxycarbonylméthoxy-3,4-dihydro-2,5,7,8-tétraméthyl-2H[1]-benzopyran-2-yl) thiocarboxamide

En procédant comme dans l'exemple 66, mais en remplaçant au stade A le bromure d'oxyde de méthyle par le bromacétate d'éthyle, on obtient le produit du titre.

### Exemple 68 : N-(4,6-diméthylpyridin-2-yl) (6-carboxyméthoxy-3,4-dihydro-2,5,7,8-tétraméthyl-2H[1]-benzopyran-2-yl) thiocarboxamide

Dissoudre 1,77 grammes (4 mmoles) du composé de l'exemple 67 dans 40 cm³ d'éthanol. Ajouter goutte à goutte 4 cm³ d'hydroxyde de sodium 2N.
Agiter pendant 2 heures, puis diluer le mélange réactionnel avec 60 cm³ d'eau, acidifier avec de l'acide acétique. Filtrer, laver à l'eau puis sécher pour obtenir le produit du titre.

### Exemple 69 : N-(4,6-diméthylpyridin-2-yl) (6-éthoxyéthoxy-3,4-dihydro-2,5,7,8-tétraméthyl-2H[1]-benzopyran-2-yl) thiocarboxamide

En procédant comme dans l'exemple 66, mais en remplaçant dans le stade A le bromure d'oxyde de méthyle par le bromure d'oxyde d'éthyle, on obtient le produit du titre.

### Exemple 70 : N-(4,6-diméthylpyridin-2-yl) (6-éthoxycarbonyloxy-3,4-dihydro-2,5,7,8-tétraméthyl-2H[1]-benzopyran-2-yl) thiocarboxamide

En procédant comme dans l'exemple 66, mais en remplaçant dans le stade A de l'exemple 66 le bromure d'oxyde de méthyle par le bromoformiate d'éthyle, on obtient le produit du titre.

### Exemple 71 : N-phényl 2-(6-acétoxy-3,4-dihydro-2,5,7,8-tétraméthyl-2H[1]-benzopyran-2-yl) thioacétamide

En procédant comme dans l'exemple 1, mais en remplaçant au stade A de l'exemple 1 l'acide(6-hydroxy-3,4-dihydro-2,5,7,8-tétraméthyl-2H[1]-benzopyran-2-yl) carboxylique par l'acide (6-hydroxy-3,4-dihydro-2,5,7,8-tétraméthyl-2H[1]-benzopyran-2-yl) acétique, on obtient le produit du titre.

### Exemple 72 : N-phényl (6-acétoxy-3,4-dihydro-5,7,8-triméthyl-2H[1]-benzopyran-2-yl) thiocarboxamide

En procédant comme dans l'exemple 1, mais en remplaçant au stade à de l'exemple 1 l'acide (6-hydroxy-3,4-dihydro-2,5,7,8-tétraméthyl-2H[1]-benzopyran-2yl) carboxylique par l'acide (6-hydroxy-3,4-dihydro-5,7,8-triméthyl-2H[1]-benzopyran-2-yl) ) carboxylique, on obtient le produit du titre.

### Exemple 73 : N-phényl (6-acétoxy-3,4-dihydro-2-méthyl-7-tert.butyl-2H[1]-benzopyran-2-yl) thiocarboxamide

En procédant comme dans l'exemple 1, mais en remplaçant au stade A de l'exemple 1 l'acide (6-hydroxy-3,4-dihydro-2,5,7,8-tétraméthyl-2H[1]-benzopyran-2-yl) carboxylique par l'acide (6-hydroxy-3,4-dihydro-2-méthyl-7-tert.butyl-2H[1]-benzopyran-2-yl) carboxylique, on obtient le produit du titre.

### Exemple 74 : N-phényl (6-hydroxy-3,4-dihydro-2-méthyl-7-tert.butyl-2H[1]-benzopyran-2-yl) thiocarboxamide

En opérant comme dans l'exemple 2 (2ème procédé), mais en remplaçant le composé de l'exemple 1 par le composé de l'exemple 73, on obtient le produit du titre.

### Exemple 75 : N-phényl (6-hexanoyloxy-3,4-dihydro-2,5,7,8-tétraméthyl-2H[1]-benzopyran-2-yl) thiocarboxamide

En procédant comme dans l'exemple 1, mais en remplaçant au stade à l'anhydride acétique par le chlorure de l'acide hexanoïque, on obtient le produit du titre.

### Exemple 76 : N-phényl (6-hydroxy-3,4-dihydro-2,5,7,8-tétraméthyl-2H[1]-benzopyran-2-yl) thiocarboxamide

### - 3ème procédé -

En procédant comme dans l'exemple 2 (2ème procédé), mais en remplaçant le composé de l'exemple 1 par le composé de l'exemple 75, on obtient le produit du titre.
Point de fusion : 150-152°C

### Exemple 77 : N-(2,6-diméthylphényl) (6-hexanoyloxy-3,4-dihydro-2,5,7,8-tétraméthyl-2H[1]-benzopyran-2-yl) thiocarboxamide

En procédant comme dans l'exemple 1, mais en remplaçant au stade A l'anhydride acétique par le chlorure de l'acide hexanoïque et au stade B l'aniline par la 2,6-diméthylaniline, on obtient le produit du titre.

### Exemple 78 : N-(2,6-diméthylphényl) (6-hydroxy-3,4-dihydro-2,5,7,8-tétraméthyl-2H[1]-benzopyran-2-yl) thiocarboxamide

### - 3ème procédé -

En procédant comme dans l'exemple 2 (2ème procédé), mais en remplaçant le composé de l'exemple 1 par le composé de l'exemple 77, on obtient le produit du titre.

### Exemple 79 : N-(4,6-diméthylpyridin-2-yl) (6-hexanoyloxy-3,4-dihydro-2,5,7,8-tétraméthyl-2H[1]-benzopyran-2-yl) thiocarboxamide

En procédant comme dans l'exemple 1, mais en remplaçant au stade A l'anhydride acétique par le chlorure de l'acide hexanoïque et au stade B l'aniline par la (4,6-diméthylpyridin-2-yl)amine, on obtient le produit du titre.

### Exemple 80 : N-(4,6-diméthylpyridin-2-yl) (6-hydroxy-3,4-dihydro-2,5,7,8-tétraméthyl-2H[1]-benzopyran-2-yl) thiocarboxamide

### - 3ème procédé -

En procédant comme dans l'exemple 2 (2ème procédé), mais en remplaçant le composé de l'exemple 1 par le composé de l'exemple 79, on obtient le produit du titre.

### Exemple 81 : N-(3,5-ditert-butyl-4-hydroxyphényl) (6-acétoxy-3,4-dihydro-2,5,7,8-tétraméthyl-2H[1]-benzopyran-2-yl) thiocarboxamide

En procédant comme dans l'exemple 1 mais en remplaçant au stade B l'aniline par la 3,5-ditert-butyl-4-hydroxyaniline, on obtient le composé du titre.

### Exemple 82 : N-(3,5-ditert-butyl-4-hydroxyphényl) (6-hydroxy-3,4-dihydro-2,5,7,8-tétraméthyl-2H[1]-benzopyran-2-yl) thiocarboxamide

En procédant comme dans l'exemple 2 mais en partant du composé de l'exemple 81, on obtient le composé du titre.
Point de fusion : 172-174°C

### Exemple 83 : 1-[(3,4-dihydro-6-acétoxy-2,5,7,8-tétraméthyl-2H-1-benzopyran-2-yl)thiocarbonyl]-4-phénylpipérazine

### STADE A : 1-[(3,4-dihydro-6-acétoxy-2,5,7,8-tétraméthyl-2H-1-benzopyran-2-yl)carbonyl]-4-phénylpipérazine

Dissoudre 3 g d'acide (6-acétoxy-3,4-dihydro-2,5,7,8-tétraméthyl-2H[1]-benzopyran-2-yl)carboxylique (stade A, exemple 1) dans 30 cm³ de benzène anhydre.

Ajouter 3 équivalents (eq) de chlorure de thionyle. Chauffer 6 h au reflux. Evaporer. Reprendre 2 fois le résidu par du benzène et évaporer. Dissoudre le résidu dans 20 cm³ de dichloroéthane. Diluer la phénylpipérazine (1,2 éq. soit 3,9 cm³) dans 20 cm³ de dichloroéthane. Verser alors à la température de la glace, goutte à goutte, la solution contenant le chlorure d'acide. Laisser revenir à température ambiante. Poursuivre l'agitation une nuit. Filtrer. Evaporer. Purifier sur colonne de gel de silice en éluant au dichlorométhane.
- Rendement : 77 %
- Point de fusion : 112-113°C

### STADE B : 1-[(3,4-dihydro-6-acétoxy-2,5,7,8-tétraméthyl-2H-1-benzopyran-2-yl)thiocarbonyl]-4-phénylpipérazine

Dissoudre 2,2 g (5,03 mmol) du composé obtenu au stade précédent dans 70 cm³ de toluène anhydre. Ajouter 2 g de réactif de Lawesson. Chauffer 8 h au reflux. Evaporer le solvant. Purifier sur colonne de gel de silice en éluant par le dichlorométhane. Isoler 1,3 g soit un rendement de 56 %.
- Point de fusion : 125-126°C

### Exemple 84 : 1-[(3,4-dihydro-6-hydroxy-2,5,7,8-tétraméthyl-2H-1-benzopyran-2-yl)thiocarbonyl]-4-phénylpipérazine

Dissoudre 1,1 g du composé précédemment obtenu dans 50 cm³ d'éthanol. Ajouter environ 15 cm³ d'une solution 1N d'hydroxyde de sodium (7 éq.). Agiter sous courant d'azote et à température ambiante pendant 2 h. Diluer le mélange dans l'eau, acidifier par l'acide acétique dilué au 1/2. Filtrer, Reprendre le résidu par du dichlorométhane. Sécher sur sulfate de sodium. Filtrer, Evaporer.
- Rendement : 90 %
- Point de fusion : 202-203°C
- Caractéristique spectrale en infra-rouge :
   ν(OH) : 3435 cm⁻¹

### Exemples 85 à 104 :

En procédant, sauf précisions contraires, comme lors des exemples 83 et 84, mais en utilisant les amines convenablement substituées, on obtient les composés des exemples suivants :

### Exemple 85 : 1-[(3,4-dihydro-6-acétoxy-2,5,7,8-tétraméthyl-2H-1-benzopyran-2-yl)thiocarbonyl]-4-(pyrid-2-yl)pipérazine

- Rendement : 47 %
- Point de fusion : 163-164°C

### Exemple 86 : 1-[(3,4-dihydro-6-hydroxy-2,5,7,8-tétraméthyl-2H-1-benzopyran-2-yl)thiocarbonyl]-4-(pyrid-2-yl)pipérazine

- Rendement : 91,6 %
- Point de fusion : 160-161°C
- Caractéristique spectrale en infra-rouge :
   ν(OH) : 3480 cm⁻¹

### Exemple 87 : 1-[(3,4-dihydro-6-acétoxy-2,5,7,8-tétraméthyl-2H-1-benzopyran-2-yl)thiocarbonyl]-4-(4-fluorophényl)pipérazine

- Rendement : 74,1 %
- Point de fusion : 134°C

### Exemple 88 : 1-[(3,4-dihydro-6-hydroxy-2,5,7,8-tétraméthyl-2H-1-benzopyran-2-yl)thiocarbonyl]-4-(4-fluorophényl)pipérazine

- Rendement : 43,7 %
- Point de Fusion : 141-142°C

### Exemple 89 : 1-[(3,4-dihydro-6-acétoxy-2,5,7,8-tétraméthyl-2H-1-benzopyran-2-yl)thiocarbonyl]-4-(4-chlorobenzhydryl) pipérazine

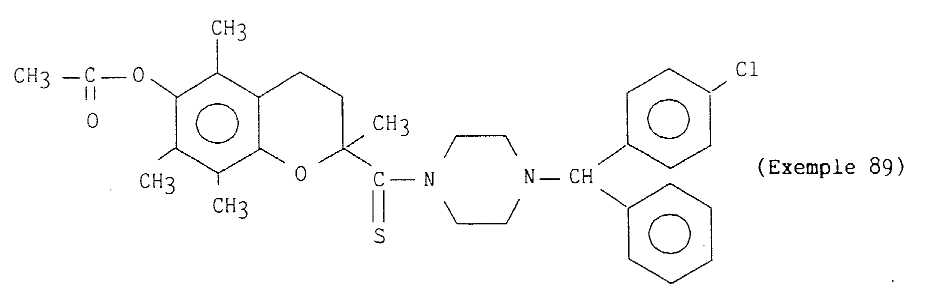

- Rendement : 73 %
- Point de fusion : 155-156°C

### Exemple 90 : 1-[(3,4-dihydro-6-hydroxy-2,5,7,8-tétraméthyl-2H-1-benzopyran-2-yl)thiocarbonyl]-4-(4-chlorobenzhydryl) pipérazine

- Rendement : 39 %
- Point de fusion 166-167°C

### Exemple 91 : 1-[(3,4-dihydro-6-acétoxy-2,5,7,8-tétraméthyl-2H-1-benzopyran-2-yl)thiocarbonyl]-4-(4-chlorophényl)pipérazine

### Exemple 92 : 1-[(3,4-dihydro-6-hydroxy-2,5,7,8-tétraméthyl-2H-1-benzopyran-2-yl)thiocarbonyl]-4-(4-chlorophényl)pipérazine

- Rendement : 39,6 %
- Point de fusion : 154-155°C (éther diisopropylique)
- Caractéristiques spectrales en infra-rouge :
   ν(OH) : 3500 cm⁻¹

### Exemple 93 : 1-[(3,4-dihydro-6-acétoxy-2,5,7,8-tétraméthyl-2H-1-benzopyran-2-yl)thiocarbonyl]-4-(2,3,4-triméthoxybenzyl) pipérazine

### Exemple 94 : 1-[(3,4-dihydro-6-hydroxy-2,5,7,8-tétraméthyl-2H-1-benzopyran-2-yl)thiocarbonyl]-4-(2,3,4-triméthoxybenzyl) pipérazine

- Rendement : 31,3 %
- Point de fusion 124-125°C (éther diisopropylique)

### Exemple 95 : 1-[(3,4-dihydro-6-acétoxy-2,5,7,8-tétraméthyl-2H-1-benzopyran-2-yl)thiocarbonyl]-4-(3,4,5-triméthoxybenzyl) pipérazine

### Exemple 96 : 1-[(3,4-dihydro-6-hydroxy-2,5,7,8-tétraméthyl-2H-1-benzopyran-2-yl)thiocarbonyl]-4-(3,4,5-triméthoxybenzyl) pipérazine

- Point de fusion 82°C (éther isopropylique)

### Exemple 97 : 1-[(3,4-dihydro-6-acétoxy-2,5,7,8-tétraméthyl-2H-1-benzopyran-2-yl)thiocarbonyl]-4-[1-hydroxy-1,1 -diphénylméthyl]pipérazine

### Exemple 98 : 1-[(3,4-dihydro-6-hydroxy-2,5,7,8-tétraméthyl-2H-1-benzopyran-2-yl)thiocarbonyl]-4-[1-hydroxy-1,1-diphénylméthyl]pipérazine

### Exemple 99 : 1-[(3,4-dihydro-6-acétoxy-2,5,7,8-tétraméthyl-2H-1-benzopyran-2-yl)thiocarbonyl]-4-(4,4'-difluorodiphénylméthyl)pipérazine

### Exemple 100 : 1-[(3,4-dihydro-6-hydroxy-2,5,7,8-tétraméthyl-2H-1-benzopyran-2-yl)thiocarbonyl]-4-(4,4'-difluorodiphénylméthyl)pipérazine

Hydrolyse du groupement acétoxy : dissoudre 0,6 g (0,001 mol) du composé de l'exemple 99 dans 400 cm³ de méthanol. Ajouter 15 cm³ d'hydroxyde de potassium. Laisser agiter 2 h sous courant d'azote. Acidifier avec de l'acide chlorhydrique 5 N. Filtrer. On recueille 0,4 g d'une poudre jaune.
- Rendement : 36,2 %
- Point de fusion : 204-205°C

### Exemple 101 : 1-[(3,4-dihydro-6-acétoxy-2,5,7,8-tétraméthyl-2H-1-benzopyran-2-yl)thiocarbonyl]-4-(4-méthoxyphényl)pipérazine

### Exemple 102 : 1-[(3,4-dihydro-6-hydroxy-2,5,7,8-tétraméthyl-2H-1-benzopyran-2-yl)thiocarbonyl]-4-(4-métheryphényl)pipérazine

- Point de Fusion : 120-121°C (éther isopropylique)

| Analyse élémentaire : | | | |
|---|---|---|---|
| | C | H | N |
| % théorique | 68,13 | 7,32 | 6,35 |
| % trouvé | 67.94 | 7,47 | 6,33 |

### Exemple 103 : 1-[(3,4-dihydro-6-acétoxy-2,5,7,8-tétraméthyl-2H-1-benzopyran-2-yl)thiocarbonyl]-4-(4,6-diméthylpyridin-2-yl)pipérazine

### Exemple 104 : 1-[(3,4-dihydro-6-hydroxy-2,5,7,8-tétraméthyl-2H-1-benzopyran-2-yl)thiocarbonyl]-4-(4,6-diméthylpyridin-2 -yl)pipérazine

- Point de fusion : 134°C

### Exemple 105 : 1-[(3,4-dihydro-2,5,7,8-tétraméthyl-6-(triméthylacétoxy)-2H-1-benzopyran-2-yl)thiocarbonyl]-4-(4-méthoxyphényl) pipérazine

### STADE A : 1-[(3,4-dihydro-6-hydroxy-2,5,7,8-tétraméthyl-2H-1-benzopyran-2-yl)carbonyl]-4-(4-méthoxyphényl)pipérazine

Disssoudre 5 g (0,017 mol) d'acide (3,4-dihydro-6-hydroxy-2H-1-benzopyran-2-yl)carboxylique dans 150 cm³ de tétrahydrofurane (THF) anhydre. Ajouter 3 g (1,1 eq. soit 0,018 mol) de carbonyldiimidazole (CDI). Laisser agiter 1 h h à température ambiante. Ajouter alors 9,5 g (0,034 mol) de 1-(4-méthoxyphényl)pipérazine )pipérazine dissoute dans 20 cm³ de THF. Laisser sous agitation 1 nuit. Evaporer. Reprendre par du dichlorométhane. Laver la phase organique avec de l'acide chlorhydrique 2 N. Sécher sur sulfate de sodium anhydre. Evaporer. Recueillir une huile. Cristalliser dans l'éther isopropylique.
- Rendement : 64,38 %
- Point de Fusion : 149-150°C (éther diisopropylique)

### STADE B : 1-[(3,4-dihydro-2,5,7,8-tétraméthyl-6-(triméthylacétoxy)-2H-1-benzopyran-2-yl)carbonyl]-4-(4-méthoxyphényl)pipérazine

Dans un ballon, dissoudre 4,4 g (0,01 mol) du composé obtenu au stade A dans 75 cm³ de pyridine anhydre. Ajouter goutte à goutte 4,99 g (4 eq. soit 0,04 mol) de chlorure d'acide triméthylacétique. Porter à 800C et maintenir sous agitation pendant 72 h. Verser le mélange sur de la glace. Extraire à l'éther. Laver la phase organique à l'eau. Sécher au sulfate de sodium anhydre. Evaporer. On obtient une huile que l'on cristallise dans l'éther diisopropylique.
- Rendement : 70 %
- Point de fusion : 141-142°C (éther diisopropylique)

### STADE C : 1-[(3,4-dihydro-2,5,7,8-tétraméthyl-6-(triméthylacétoxy)-2H-1-benzopyran-2-yl)thiocarbonyl]-4-(4-méthoxyphényl)pipérazine

On procéde comme au stade B de l'exemple 83 pour obtenir le composé attendu.
- Rendement : 64,7 %
- Point de fusion : 101-102°C (éther diisopropylique)

### Exemple 106 : 1-[(3,4-dihydro-6-acétoxy-2,5,7,8-tétraméthyl-2H-1-benzopyran-2-yl)thiocarbonyl]-4-(1,1-diphényl-1-acétoxyméthyl)pipéridine

### STADE A : 1-[(6-acétoxy-3,4-dihydro-2,5,7,8-tétraméthyl-2H-1-benzopyran-2-yl)carbonyl]-4-(1,1-diphényl-1-hydroxyméthyl)pipéridine

Dissoudre 2,7 g (0,0094 mol) d'acide (3,4-dihydro-6-acétoxy-2H-1-benzopyran-2-yl)carboxylique dans 150 cm³ de benzène anhydre. Ajouter goutte à goutte 4 cm³ de chlorure de thionyle. Laisser 4 h au reflux. Evaporer le solvant. Reprendre le résidu par du benzène puis évaporer à nouveau. Recommencer 2 fois l'opération. Diluer le résidu huileux dans 30 cm³ de dichloroéthane anhydre. Verser goutte à goutte la solution de chlorure d'acide sur une suspension de 6 g (0,22 mol) de 1,1-diphényl-1-(pipéridin-4-yl)méthanol dans 100 cm³ de dichloroéthane. Laisser l'agitation une nuit. Filtrer, évaporer, obtenir une huile. Purifier sur colonne de silice en éluant avec du dichlorométhane. Recueillir une huile. Faire cristalliser dans l'éther diisopropylique.
- Rendement : 77,5 %
- Point de Fusion : 188-189°C (éther isopropylique)

### STADE B : 1-[(6-acétoxy-3,4-dihydro-2,5,7,8-tétraméthyl-2H-1-benzopyran-2-yl)carbonyl]-4-(1,1-diphényl-1-acétoxyméthyl)pipéridine

A la température de la glace, verser dans une solution de 30 cm³ de chlorure d'acétyle 0,5 g (0,002 mol) du composé obtenu au stade précédent. Laisser agiter une nuit à température ambiante. Evaporer. Reprendre le résidu huileux par du benzène anhydre. Evaporer. Obtenir une huile. Purifier sur colonne de silice neutre en éluant au dichlorométhane.

### STADE C : 1-[(6-acétoxy-3,4-dihydro-2,5,7,8-tétraméthyl-2H-1-benzopyran-2-yl)thiocarbonyl]-4-(1,1-diphényl-1-acétoxyméthyl)pipéridine

A une solution de 400 mg (0,006 mol) du composé obtenu au stade précédent dans 100 cm³ de toluène anhydre, ajouter 0,3 g (0,72 mmol) de réactif de Lawesson. Laisser pendant 12 h au reflux. Evaporer. Purifier l'huile obtenue sur colonne de silice en éluant au dichlorométhane. Recueillir une huile jaune.
- Rendement : 67 %

### Exemple 107 : 1-[(3,4-dihydro-6-hydroxy-2,5,7,8-tétraméthyl-2H-1-benzopyran-2-yl)thiocarbonyl]-4-(1,1-diphénylméthylidényl) pipéridine

A une solution de 0,4 g (0,7 mol) du composé obtenu à l'exemple 106 dans 30 cm³ d'éthanol, ajouter 5 cm³ de NaOH 1M. Laisser agiter 2 h sous courant d'azote. Ajouter 50 cm³ d'eau. Acidifier avec de l'acide chlorhydrique 2M. Filtrer.
- Rendement : 83,3 %

### Exemple 108 : N-phényl-(3,4-dihydro-6-acétoxy-3,5,7,8-tétraméthyl-2H-1-benzopyran-2-yl)thiocarboxamide

En procédant comme dans l'exemple 1 mais en remplaçant au stade A l'acide (6-hydroxy-3,4-dihydro-2,5,7,8-tétraméthyl-2H-1-benzopyran-2-yl)carboxylique par l'acide (6-hydroxy-3,4-dihydro-3,5,7,8-tétraméthyl-2H-1-benzopyran-2-yl)carboxylique, on obtient le composé du titre.

### Exemple 109 : N-phényl-(3,4-dihydro-6-hydroxy-3,5,7,8-tétraméthyl-2H-1-benzopyran-2-yl)thiocarboxamide

En procédant comme dans l'exemple 2 mais en partant du composé de l'exemple 108, on obtient le produit du titre.
- Rendement : 45 %
- Point de fusion : 175°C

### Exemple 110 : 1-[(3,4-dihydro-6-acétoxy-2,5,7,8-tétraméthyl-2H-1-benzopyran-2-yl)thiocarbonyl]-4-(tert-butoxycarbonyl) pipérazine

### STADE A : 1-[(3,4-dihydro-6-acétoxy-2,5,7,8-tétraméthyl-2H-1-benzopyran-2-yl)carbonyl]pipérazine

Dissoudre 2,7 g (0,009 mol) d'acide (3,4-dihydro-6-acétoxy-2,5,7,8-tétraméthyl-2H-1-benzopyran-2-yl)carboxylique dans 200 cm³ de dichloroéthaneanhydre. Ajouter 1 eq. de carbonyldiimidazole (0,009 mol), soit 1,5 g. Laisser agiter 1 h puis ajouter 7,5 g de pipérazine (0,009 mol) dissoute dans 200 cm³ de dichloroéthane. Purifier sur colonne de silice l'huile obtenue après évaporation en éluant avec un mélange (88 CH₂Cl₂ ; 10 éthanol, 2 NH₄ OH). Cristalliser dans l'éther isopropylique.
- Rendement : 51 %

### STADE B : 1-[(3,4-dihydro-6-acétoxy-2,5,7,8-tétraméthyl-2H-1-benzopyran-2-yl)carbonyl]-4-(tert-butoxycarbonyl)pipérazine

Ajouter 1 g (1,1 eq., soit 0,004 mol) de di-tert-butyl pyrocarbonate à une solution de 1,4 g (0,03 mol) du composé obtenu au stade précédent, et de 0,75 cm³ de triéthylamine dans un mélange à 50 % d'eau et de dioxane. Laisser agiter 2 h à température ambiante. Ajouter de l'eau et de l'acétate d'éthyle distillé. Sécher la phase organique avec du sulfate de sodium anhydre. Evaporer, purifier sur colonne en éluant avec du chlorure de méthylène. On obtient une huile transparente.
- Rendement : 60 %

### STADE C : 1-[(3,4-dihydro-6-acétoxy-2,5,7,8-tétraméthyl-2H-1-benzopyran-2-yl)thiocarbonyl]-4-(tert-butyloxycarbonyl)pipérazine

En procédant comme dans le stade B de l'exemple 83, on obtient le composé attendu.

### Exemple 111 : 1-[(6-acétoxy-3,4-dihydro-2,5,7,8-tétraméthyl-2H-1-benzopyran-2-yl)thiocarbonyl]pipérazine

Dissoudre 1,1 g du composé obtenu à l'exemple 110 (2,3.10⁻³ mol) dans 30 cm³ d'acide trifluoroacétique. Laisser agiter 2 h. Evaporer, reprendre par du chlorure de méthylène. Ajouter 4 cm³ de triéthylamine. Agiter 3 h. Laver la phase organique à l'eau. Sécher sur sulfate de sodium anhydre. Evaporer. On obtient une substance glacée jaune.
- Rendement : 81 %

### Exemple 112 : 1-[(3,4-dihydro-6-hydroxy-2,5,7,8-tétraméthyl-2H- 1-benzopyran-2-yl)thiocarbonyl]-4-[2,6-di(pyrrolidin-1-yl)pyrimidin-4-yl]pipérazine

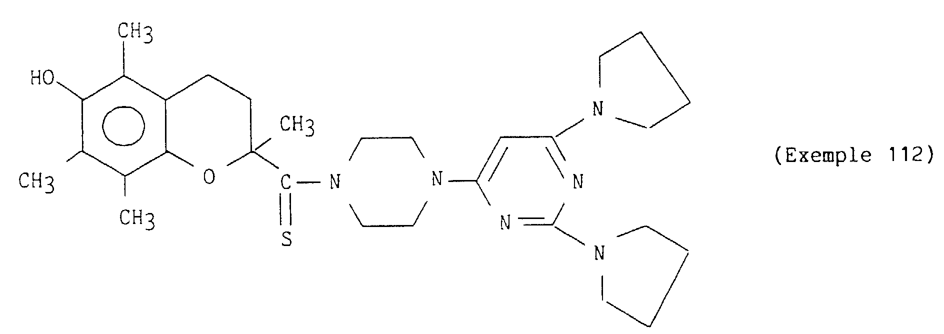

Dissoudre 0,8 g (1,8.10⁻³ mol) du composé obtenu à l'exemple 111 et 1,5 g (5,6.10⁻³ mol) de 4-chloro-2,6-di(pyrrolidin-1-yl)pyrimidine dans 50 cm³ de pyridine anhydre. Chauffer 2 h au reflux. Evaporer. Reprendre par du dichlorométhane. Laver la phase organique avec de l'acide chlorhydrique 1N. Purifier l'huile obtenue après évaporation sur colonne de silice en éluant avec du dichlorométhane. On recueille une huile jaune.
- Rendement : 25 %
- Point de fusion : 168°C
- Caractéristique spectrale en infra-rouge :
   ν(OH) : 3440 cm⁻¹

### Exemples 113 à 122 :

En utilisant les procédés précédemment décrits, on peut également obtenir à partir des matières premières appropriées les composés des exemples suivants :

### Exemple 113 : N-phényl-(6-acétoxy-3,4-dihydro-3-éthyl-5,7,8-triméthyl-2H-1-benzopyran-2-yl)thiocarboxamide

### Exemple 114 : N-phényl-(6-hydroxy-3,4-dihydro-3-éthyl-5,7,8-triméthyl-2H-1-benzopyran-2-yl)thiocarboxamide

### Exemple 115 : 1-[(6-acétoxy-3,4-dihydro-3-éthyl-5,7,8-triméthyl-2H-1-benzopyran-2-yl)thiocarbonyl]-4-(4-fluorophényl)pipérazine

### Exemple 116 : 1-[(6-hydroxy-3,4-dihydro-3-éthyl-5,7,8-triméthyl-2H-1-benzopyran-2-yl)thiocarbonyl]-4-(4-fluorophényl)pipérazine

### Exemple 117 : 1-[(6-acétoxy-3,4-dihydro-7-tert-butyl-2H-1-benzopyran-2-yl)thiocarbonyl]-4-(4-fluorophényl)pipérazine

### Exemple 118 : 1-[(6-hydroxy-3,4-dihydro-7-tert-butyl-2H-1-benzopyran-2-yl)thiocarbonyl]-4-(4-fluorophényl)pipérazine

### Exemple 119 : 1-[(6-acétoxy-3,4-dihydro-5,7,8-triméthyl-2H-1-benzopyran-2-yl)thiocarbonyl]-4-(4-fluorophényl)pipérazine

### Exemple 120 : 1-[(6-hydroxy-3,4-dihydro-5,7,8-triméthyl-2H-1-benzopyran-2-yl)thiocarbonyl]-4-(4-fluorophényl)pipérazine

### Exemple 121 : 1-[(6-acétoxy-3,4-dihydro-3,5,7,8-tétraméthyl-2H-1-benzopyran-2-yl)thiocarbonyl]-4-(4-fluorophényl)pipérazine

### Exemple 122 : 1-[(6-hydroxy-3,4-dihydro-3,5,7,8-tétraméthyl-2H-1-benzopyran-2-yl)thiocarbonyl]-4-(4-fluorophényl)pipérazine

### Exemple 123 : N-(4-guanidinobut-1-yl) (3,4-dihydro-6-hydroxy-2,5,7,8-tétraméthyl-2H-1-benzopyran-2-yl)thiocarboxamide

### Exemple 124 : {1-[(3,4-dihydro-6-hydroxy-2,5,7,8-tétraméthyl-2H -1-benzopyran-2-yl)thiocarbonyl]-4-hydroxypyrrolidin-2-yl}carboxylate d'éthyle

### Exemple 125 : Acide {1-[(3,4-dihydro-6-hydroxy-2,5,7,8-tétraméthyl-2H -1-benzopyran-2-yl)thiocarbonyl]-4-hydroxypyrrolidin-2-yl}carboxylique

### ETUDE PHARMACOLOGIQUE DES COMPOSES DE L'INVENTION

**(Les composés sont comparés avec le composé le plus proche de l'art antérieur qui est l'exemple 102 de la demande WO 88/08424)**

### Exemple A : ETUDE DE L'ACTIVITE ANTIPEROXYDANTE

L'action des composés de l'invention, susceptibles de pièger les radicaux ^{.}OH a été étudiée d'une part, sur la peroxydation spontanée des lipides et d'autre part, sur la peroxydation induite par le système Fe²⁺ - ascorbate (10 µM - 250 µM), et ce sur des homogénats de cerveaux de rats.

Lors de la mesure de la peroxydation lipidique spontanée, les homogénats de cerveau de rats sont placés en présence ou en absence des composés à tester durant 60 minutes à 37°C. La réaction est arrêtée à 0°C et le dosage du malondialdéhyde est effectué à l'aide d'acide thiobarbiturique par la méthode de YAGI, K (1976) Biochem. Med, 15, 212 - 216. La peroxydation lipidique est déterminée par les substances réagissant avec l'acide thiobarbiturique exprimées en nanomoles de malondialdéhyde.

Lors de la mesure de la peroxydation lipidique induite, la méthodologie est identique à la précédente à l'exception de l'addition à l'homogénat du système inducteur de radicaux : Fe²⁺ - ascorbate. Les substances de référence sont le probucol et la vitamine E.

Les concentrations des composés testés inhibant de 50 % la peroxydation du substrat ou l'activité antioxydante des composés de l'invention à une concentration de 10⁻⁵ M sont calculées.

Il est apparu que certains composés de l'invention possèdent une activité antiperoxydante particulièrement intense puisque supérieure d'un facteur 100 à celle du composé le plus proche de l'art antérieur. Ce résultat très intéressant se produit que la peroxydation soit spontanée ou induite par un système chimique.

**TABLEAU A :**

| **ETUDE DE L'ACTIVITE ANTIPEROXYDANTE DES COMPOSES DE L'INVENTION A 10**^{**-5**} **M** | | |
|---|---|---|
| **Composé (N° EXEMPLE)** | **INHIBITION DE LA PEROXYDATION PAR RAPPORT AU TEMOIN** | |
| | **PEROXYDATION SPONTANEE** | **PEROXYDATION INDUITE** |
| 2 | 100 | 100 |
| 4 | 98 | 100 |
| 26 | 99 | 100 |

### Exemple B : ETUDE DU POUVOIR PROTECTEUR DE L'OXYDATION DES LDL

La capacité des composés de l'invention à diminuer les proportions de LDL oxydées a été mesurée de la façon suivante. On réalise une incubation de 24 heures regroupant des LDL natives, un système Cu²⁺ générateur de radicaux libres et les composés à tester.

Les résultats sont obtenus après analyse du milieu par une technique chromatographique haute performance : la FPLC (Fast Protein Liquid Chromatography). Le pouvoir protecteur du composé testé est déterminé après comparaison du chromatogramme obtenu avec celui du témoin positif de référence : le probucol.

Il apparaît clairement que les composés de l'invention ont un pouvoir protecteur très important et significativement supérieur à celui du composé le plus proche de l'art antérieur.

### Exemple C : ETUDE DE L'ACTIVITE INHIBITRICE DES COMPOSES DE FORMULE (I) UTILISES SELON L'INVENTION SUR LA SYNTHESE DES EICOSANOIDES

### 1) ETUDE DE L'ACTIVITE INHIBITRICE SUR LA SYNTHESE DE EICOSANOIDES ISSUS DE LA CYCLOOXYGENASE

Le but de cette étude est de mesurer l'activité inhibitrice des molécules utilisées selon l'invention sur la sécrétion de prostaglandine E₂ (PGE₂), un des principaux eicosanoïdes produits par la cyclooxygénase des granulocytes humains stimulés par l'ionophore calcique A23187.

### PROTOCOLE :

- **Isolement des granulocytes humains :**
   Du sang veineux humain provenant de donneurs sains n'ayant pas pris de médicaments depuis 2 semaines est prélevé dans des tubes en polypropylène contenant 1 volume d'anti-coagulant (acide citrique 2,73 %, citrate de sodium 4,48 %, glucose 2 %) pour 10 volumes de sang.
   Dans l'heure suivant le prélèvement, du dextran à 6 % est ajouté au sang (0,3 cm³/cm³ de sang). Après une incubation de 30 min à 37° C, le plasma riche en globules blancs est centrifugé à la vitesse de 100 g pendant 5 min à 4° C.
   Le culot est remis en suspension dans 3 cm³ de NH₄Cl à 0,83 % (pour lyser les globules rouges contaminants) et centrifugé à une vitesse de 100 g pendant 5 min à 4° C.
   Le culot riche en globules blancs mono- et polynuclés est récupéré dans 5 cm³ de tampon phosphate (pH 7,4) de composition suivante (en mM) : 137 : NaCl ; 2,68 : KCl ; 8,1 : Na₂HPO₄ ; 1,47 : KH₂PO₄ ; 0,9 : CaCl₂ ; 0,5 : MgCl₂ et déposé sur 3 cm³ d'une solution de ficoll type 400 à une densité de 1,077.
   Après centrifugation à une vitesse de 420 g pendant 30 min à 4° C, le culot riche en granulocytes est remis en suspension dans 5 cm³ de tampon phophate et centrifugé à une vitesse de 100 g, 5 min à 4° C. Finalement, les granulocytes sont comptés et la densité est ajustée à 3 × 10⁶ cellules/cm³ de tampon phosphate.
- **Stimulation des granulocytes par l'ionophore calcique A23187 :**
   Les cellules (3 × 10⁶ cellules/cm³) sont préincubées à 37° C pendant 15 min en absence ou en présence des produits à tester à la concentration désirée. Puis les cellules sont stimulées pendant 15 min à 37° C avec le A23187 à 5 × 10⁻⁶ M (solution mère à 10⁻² M dans le DMSO). Le taux basal est mesuré à partir de cellules ne recevant ni produits à tester, ni A23187.
   La réaction est stoppée dans la glace et le surnageant est récupéré après centrifugation à une vitesse de 250 g pendant 5 min à 4° C.
- **Dosage de PGE**_{**2**} **:**
   La quantité de PGE₂ produite est mesurée par un test radioimmunologique (RIA). Une gamme d'étalonnage est effectuée dans les mêmes conditions avec des concentrations communes de PGE₂.
- **Résultats :**
   Les composés de formule (I) utilisés selon l'invention montrent une activité inhibitrice de la synthèse des eicosanoïdes issus de la cyclooxygénase très supérieure à celle du probucol.

### 2) ETUDE DE L'ACTIVITE INHIBITRICE SUR LA SYNTHESE DES EICOSANOIDES ISSUS DE LA LIPOXYGENASE

L'activité inhibitrice sur la synthèse des eicosanoïdes des composés de formule (I) utilisés selon l'invention est mesurée sur cellules polynucléaires humaines lavées, en présence ou en absence du composé à tester, après activation de ces cellules par le calcium (ionophore calcique A 23187).

La production du principal eicosanoïde, issu de la lipoxygénase, produit par les cellules polynucléaires humaines : le leucotriène B₄, (LTB₄) est mesurée par un test radioimmunologique.

Les composés de formule (I) utilisés selon l'invention montrent une activité inhibitrice de la synthèse des eicosanoïdes issus de la lipoxygénase très supérieure à celle du probucol.

**TABLEAU B :**

| **ACTIVITE INHIBITRICE DES COMPOSES DE L'INVENTION SUR LA BIOSYNTHESE DES EICOSANOIDES ISSUS DE LA LIPOXYGENASE** | | |
|---|---|---|
| **Composé (N° EXEMPLE)** | **INHIBITION DE LA SYNTHESE DU LTB**_{**4**} | |
| | **% D'INHIBITION A 10**^{**-5**} **M** | **CONCENTRATION INHIBANT LA SYNTHESE DE 50 % (IC**_{**50**} **en M)** |
| 2 | > 99% | 0,3 |
| 4 | > 99% | 0,5 |
| 6 | > 99% | 0,6 |
| 8 | > 99% | |
| 26 | > 99% | 0,5 |
| 84 | > 99% | 0,3 |
| 86 | > 99% | 0,2 |
| 88 | > 99% | 0,1 |
| 92 | > 99% | 0,2 |
| 94 | > 99% | 0,3 |
| 98 | > 99% | 1,3 |
| | | |

### CONCLUSION :

Les études 1 et 2 de l'exemple C montre que les composés utilisés selon l'invention possède une intense activité inhibitrice sur la synthèse des eicosanoïdes.

### Exemple D : ETUDE DU POUVOIR PROTECTEUR DES COMPOSES DE L'INVENTION SUR LE PH INTRACELLULAIRE

Le pouvoir protecteur du pH intracellulaire des composés de l'invention a été testé sur les transporteurs Cl-/HCO₃- (régulateurs du pH intracellulaire) de cardiocytes en culture.

### METHODE :

### - Cardiocytes en culture (Eur. J. Pharmacol. 1991, vol. 205, pp 29-34)

### Cellules :

Nous avons utilisé une lignée de myoblastes cardiaques de rat (H₉C₂), commercialisée par ATCC (Rockville, Maryland. USA). Dans ces cellules, la sortie unidirectionnelle de bicarbonate est catalysée dans sa plus grande partie par l'échangeur CL⁻/HCO₃⁻ sodium indépendant et l'entrée unidirectionnelle de bicarbonate est catalysée, dans sa plus grande partie, par le transporteur de bicarbonate couplé au Na⁺ extracellulaire (échangeur Cl⁻/HCO₃⁻ sodium-dépendant). Les variations de pH intracellulaires concomitantes peuvent être suivies par spectrofluorimétrie. Les cellules ont été cultivées en flacons de culture de 75 cm². A chaque passage, les cellules ont été détachées par traitement à la trypsine. Les cellules ont été reprises avec un volume ad hoc de milieu de culture frais et ensemencées sur des lamelles de verre stériles de 3,15 cm². Les cellules sont utilisées à confluence, de 1 à 2 jours plus tard.

### Détermination du pH intracellulaire à l'aide du BCECF (2', 7'-bis(carboxyéthyl)-(5,6)-carboxyfluorescéine) :

Nous avons utilisé une molécule dérivée de la fluorescéine, le BCECF, dont la fluorescence dépend de son état de protonation : cette molécule présente la particularité de fluorescer à 525 nm, après avoir été excitée à 508 nm, et ce d'autant moins que le pH est bas.

La fluorescence du BCECF est calibrée, en terme de pH intracellulaire, dans un milieu dont la concentration en K⁺ est égale à la concentration en K⁺ intracellulaire et en présence de 10 µM de nigéricine (ionophore qui échange K⁺ pour H⁺ et qui assure l'égalité des pH intra- et extra- cellulaires). Des quantités connues et croissantes d'acide MOPS 1 M (acide 4-morpholinopropanesulfonique) sont ajoutées au milieu, et le pH résultant et la fluorescence sont mesurés.

### Charge en BCECF :

Après retrait du milieu de culture initial, et deux lavages avec du milieu de Ringer, les lamelles sont incubées dans 1 cm³ de milieu de Ringer contenant du BCECF-AM, la forme estérifiée à 37°C. Le milieu Ringer a la composition suivante (mM) : NaCl 145, KCl 5, MgCl₂ 1, CaCl₂ 1, MOPS-TRIS 10 (pH 7.4), glucose 5. Ensuite, le milieu est remplacé par du milieu de Ringer ne contenant pas du BCECF-AM et les cellules sont incubées pendant 10 minutes à 37°C afin de permettre une meilleure désestérification du marqueur. Enfin, les cellules sont incubées pendant 10 minutes à température ambiante (25°C) soit dans le milieu de Ringer, soit dans un milieu similaire (milieu Bicarbonate) où 25 mM de Na⁺Cl⁻ ont été substituées par 25 mM de Na⁺HCO₃⁻ (à pH constant). Cette dernière étape permet aux cellules de s'adapter à la température expérimentale. Le cas échéant, elle permet l'équilibration du pH cytosolique en fonction du bicarbonate. Pour la détermination de l'entrée de bicarbonate, les cellules sont préincubées en absence de bicarbonate et les mesures expériementales sont réalisées en présence de bicarbonate (phase d'entrée de bicarbonate = alcalinisation). A l'inverse pour la détermination de la sortie de bicarbonate, les cellules sont préincubées en présence de bicarbonate et les mesures expérimentales sont réalisées en absence de bicarbonate (phase de sortie du bicarbonate = acidification). Il est à noter que les expériences relatives à la sortie de bicarbonate sont réalisées en présence de 2 mM d'amiloride destinée à réduire les compensations de pH qui se produisent lorsque l'échangeur [Na⁺/H⁺] est activé.

### Mesure de la fluorescence :

Les lamelles sont disposées verticalement sur un portoir qui est placé dans une cuve de fluorimétrie contenant 2 cm³ du milieu expérimental (milieu de Ringer ou milieu Bicarbonate). La fluorescence est mesurée à 525 nm, après excitation à 508 nm, sur un spectrofluorimètre Shimadzu RF 5000. Les fentes d'excitation et d'émission sont de 5 nm.

Les mesures sont effectuées à température ambiante (25°C) toutes les 2 secondes. Une expérience type (une lamelle) dure environ 15 minutes. Le cas échéant, la calibration est réalisée à la fin de l'expérience, par passage en milieu K⁺ avec nigéricine et ajouts successifs d'aliquots acides (5 µl de MOPS 1M dans un volume de 2 cm³).

Dans ces expériences, l'autofluorescence des cellules et le bruit de fond sont négligeables (< 1 % du signal total, soit un rapport signal/bruit > 100).

### RESULTATS :

Les résultats de cette étude montre que les composés de l'invention sont de très puissants inhibiteurs de la sortie de bicarbonate des cardiocytes. La sortie de bicarbonate des cardiocytes est catalysée par l'échangeur Cl⁻/HCO₃⁻ indépendant du sodium, et non par celui dépendant du sodium.
Par contre, il apparaît par contre que les composés de l'invention sont de très faibles inhibiteurs de l'entrée de bicarbonate, qui est essentiellement catalysée par l'échangeur CL⁻/HC03⁻ sodium-dépendant.

**En conclusion,** les composés de l'invention sont de puissants inhibiteurs de l'échangeur Cl⁻/HCO₃⁻ indépendant du sodium, propriété qui se traduit par une rétention cellulaire du bicarbonate. Au niveau du tissu ischémié, ceci est avantageux pour neutraliser l'acidification cellulaire et les traumatismes ioniques et métaboliques associés à cette acidification.

### Exemple E : ETUDE DE LA TOXICITE AIGUE

La toxicité aiguë a été appréciée après administration orale à des lots de 3 souris (20 ± 2 grammes) de doses croissantes (0,1 - 0,25 - 0,50 - 0,75 - 1g/kg) de composés de formule (I). Les animaux ont été observés à intervalles réguliers au cours de la première journée et quotidiennement pendant les 2 semaines suivant le traitement.

Il apparaît que les composés de formule (I) sont totalement atoxiques. Aucun décès n'est observé après une administration d'une dose de 1g.kg⁻¹. On ne constate pas de troubles après adminsitration de cette dose.

### Exemple F : COMPOSITION PHARMACEUTIQUE : COMPRIMES

Comprimés dosés à 10 mg de N-phényl (6-hydroxy-3,4-dihydro-2,5,7,8-tétraméthyl-2H[1]-benzopyran-2-yl) thiocarboxamide

| Formule de préparation pour 1 000 comprimés : | |
|---|---|
| N-phényl (6-hydroxy-3,4-dihydro-2,5,7,8-tétraméthyl-2H[1]-benzopyran-2-yl) thiocarboxamide | 10g |
| Amidon de blé | 15g |
| Amidon de maïs | 15g |
| Lactose | 65g |
| Stéarate de Magnesium | 2g |
| Silice | 1g |
| Hydroxypropylcellulose | 2g |

## Revendications

1. Composés de formule (I) : 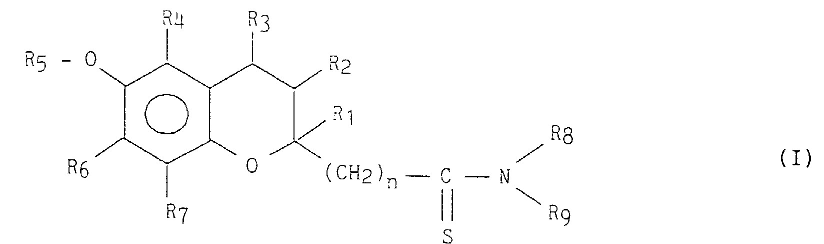 dans laquelle :
n représente un nombre entier égal à 0 ou 1,
R₁, R₂, R₃, R₄, R₆ et R₇, identiques ou différents, représentent chacun indépendamment l'un de l'autre un atome d'hydrogène ou un radical alkyl inférieur Rₐ-, où Ra représente un groupement alkyle linéaire ou ramifié de 1 à 8 atomes de carbone,
R₅ représente :
- un atome d'hydrogène,
- un groupement alkyle inférieur Rₐ-,
- un groupement acyle inférieur Rₐ-CO-,
- un groupement alcoxyalkyle de forme Rₐ-O-R_{b}-,
- un groupement alcoxycarbonyle de forme Rₐ-O-CO-,
- un groupement alcoxycarbonylalkyle de forme Rₐ-O-CO-R_{b}-,
- un groupement carboxylalkyle de forme HOOC-Rₐ-,
où Rₐ et R_{b}, identiques ou différents, représentent chacun indépendamment l'un de l'autre, un radical alkyle linéaire ou ramifié comportant de 1 à 8 atomes de carbone,
R₈ et R₉
- soit forment ensemble avec l'atome d'azote qui les porte un groupement choisi parmi :
. pipérazine,
. pipérazine substituée,
. pipéridine,
. pipéridine substituée,
. pyrrolidine,
. pyrrolidine substituée,
. morpholine,
. morpholine substituée par un ou plusieurs radicaux alkyles,
. tétrahydropyridine,
. thiomorpholine,
. azaspirane de 5 à 12 chaînons,
. azaspirane de 5 à 12 chaînons substitué par un ou plusieurs radicaux alkyles ou groupement oxo,
. azacycloalkyle mono ou bicyclique de 7 à 12 chaînons incluant éventuellement dans son squelette de 1 à 2 hétéroatomes supplémentaires choisis parmi oxygène, soufre, et azote,
. azacycloalkyle mono ou bicyclique de 7 à 12 chaînons, substitué par un ou plusieurs radicaux alkyles ou groupements oxo, incluant éventuellement 1 à 2 hétéroatomes supplémentaires choisis parmi oxygène, soufre, et azote,
. un groupement -NH-(CH₂)ₖ-NH₂ dans lequel k représente un nombre entier égal à 2, 3, ou 4,
. et un groupement -NH-(CH₂)ₖ-NH₂ substitué dans lequel k est tel que défini précédemment,
étant entendu que le terme "substitué(e)" affectant ci-dessus les groupements pipérazine, pipéridine, pyrrolidine, et -NH-(CH₂)ₖ-NH₂ signifie que ces groupements peuvent être substitués par un ou plusieurs atomes d'halogène, radicaux hydroxyles, radicaux carboxyles, radicaux R₁₀, ou radicaux 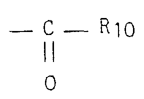 avec R₁₀ étant choisi parmi :
. alkyle,
. alkoxy,
. alcényle,
. -(CH₂)ₙ-R₁₁ ou 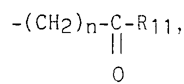 où n représente 0 ou un nombre entier de 1 à 5 et où R₁₁ représente un radical choisi parmi phényle, benzhydryle, 1,1-diphénylméthylidényle, thiényle, pyrrolyle, pyrrolidinyle, furyle, pyrimidinyle, pyridyle, benzodioxolyle, benzodioxanyle, naphtyle, quinoléinyle, isoquinoléinyle, cycloalkyle, et dicycloalkylméthyle ; le terme "cycloalkyle" représentant un groupement mono- ou bi-cyclique de 3 à 12 chaînons, ces radicaux R₁₀ pouvant eux-mêmes être substitués par un ou plusieurs radicaux choisis parmi halogène, trifluorométhyle, oxo, carboxyle, hydroxyle, alkyle, alkoxy, halogénoalkoxy, acétyle, et pyrrolidinyle,
- soit R₈ et R₉, identiques ou différents, représentent chacun indépendamment l'un de l'autre :
. un atome d'hydrogène,
. un groupement alkyle inférieur Rₐ- ou un groupement alkyle inférieur Rₐ- substitué,
. un groupement alcényle inférieur ou un groupement alcényle inférieur substitué, où le groupement alcényle représente un hydrocarbure insaturé, linéaire ou ramifié, comprenant 2 à 8 atomes de carbone,
. un groupement A-(CH₂)ₘ- ou un groupement A-(CH₂)ₘ- substitué, où m est un nombre entier égal à 0,1, ou 2 et A représente un groupement cycloalkyle comportant p atomes de carbone avec p étant un nombre entier de 3 à 7,
. un groupement alcoxyalkyle de forme Rₐ-O-R_{b}- ou un groupement alcoxyalkyle de forme Rₐ-O-R_{b}- substitué, où Rₐ et R_{b}, identiques ou différents, représentent un radical alkyle inférieur, linéaire ou ramifié, de 1 à 8 atomes de carbone,
. un groupement alcoxycarbonylalkyle de forme Rₐ-O-CO-R_{b}- ou un groupement alcoxycarbonylalkyle de forme Rₐ-O-CO-R_{b}- substitué, avec Rₐ et R_{b} tels que définis ci-dessus,
. un groupement B-(CH₂)_{q}- ou un groupement B-(CH₂)_{q}- substitué, où q est un nombre entier égal à 0, 1, 2, ou 3 et B représente un radical naphtalène, 1,3-dioxane , pyrane, ou benzopyrane,
. un groupement E-(CH₂)_{q}- ou un groupement E-(CH₂)_{q}- substitué, avec q tel que défini précédemment et E représentant un groupement azaspirane ou azacycloalkyle, non substitués ou substitués, tel que définis précédemment,
. un groupement phényl-(CH₂)_{q}- ou un groupement phényl-(CH₂)_{q}- substitué, avec q tel que défini ci-dessus,
. un groupement hétéroaryl-(CH₂)_{q}- ou un groupement hétéroaryl-(CH₂)_{q}- substitué, avec q tel que défini ci-dessus et où l'hétéroaryle est choisi parmi :
furane, quinoléine, isoquinoléine, pyridine, thiophène, thiazole, isothiazole, oxazole, isoxazole, naphtyridine, benzofurane, β-carboline, ou Υ-carboline,
. un groupement guanidino ou amidino, non substitué ou substitué par un ou plusieurs alkyles linéaires ou ramifiés de 1 à 6 atomes de carbone,
. un des radicaux D₁ à D₄ suivants : 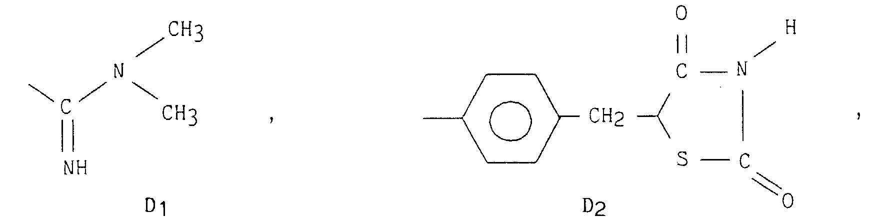 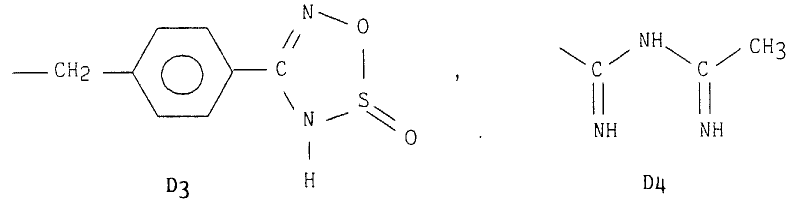
étant entendu que
lors de cette description de la formule générale (I), le terme "substitué" affectant les groupements tels que définis précédemment : alkyle inférieur Ra-, alcényle inférieur, A-(CH₂)ₘ-, alcoxyalkyle Rₐ-O-R_{b}-, alcoxycarbonylalkyle Rₐ-O-CO-R_{b}-, B-(CH₂)_{q}-, phényl-(CH₂)_{q}-, hétéroaryl-(CH₂)_{q}-, signifie, lorsque cela n'est pas précisé, que ces groupements peuvent être substitués par un ou plusieurs radicaux, identiques ou différents, et représentant chacun indépendamment l'un de l'autre :
un groupement alkyle inférieur R_{c}-,
alcoxy inférieur R_{c}-O-,
acyle inférieur R_{c}-CO-,
trifluorométhyle,
carboxyle,
hydroxyle,
oxo,
guanidino,
amidino,
ou un atome d'halogène,
où R_{c} représente un groupement alkyle linéaire ou ramifié de 1 à atomes de carbone,
leurs isomères optiques, sous forme pure ou sous forme de mélange,
ainsi que, le cas échéant, leurs sels d'addition à un acide ou à une base pharmaceutiquement acceptable.

2. Composés selon la revendication 1 dans lesquels n représente O, R₈ représente un atome d'hydrogène et R₉ représente un groupement phényle ou phényle substitué par un ou plusieurs radicaux choisis parmi alkyle inférieur Rc-, alcoxy inférieur Rc-O-, acyle inférieur Rc-CO-, trifluorométhyle, carboxyle, hydroxyle, et halogène, avec Rc désignant un groupement alkyle linéaire ou ramifié de 1 à 6 atomes de carbone, leurs isomères optiques, sous forme pure ou sous forme de mélange, ainsi que leurs sels d'addition à un acide ou à une base pharmaceutiquement acceptable.

3. Composés selon la revendication 1 dans lesquels R₅ représente un atome d'hydrogène,
leurs isomères optiques, sous forme pure ou sous forme de mélange, et leurs sels d'addition à un acide ou à une base pharmaceutiquement acceptable.

4. Composés selon la revendication 1 dans lesquels R₈ et R₉ forment ensemble avec l'atome d'azote qui les porte une pipérazine non substituée ou substituée,
leurs isomères optiques,
et leurs sels d'addition à un acide ou une base pharmaceutiquement acceptable.

5. Composé selon la revendication 1 qui est le N-phényl (6-hydroxy-3,4-dihydro-2,5,7,8-tétraméthyl-2H[1]-benzopyran-2-yl) thiocarboxamide, ses isomères optiques, et ses sels d'addition à un acide ou à une base pharmaceutiquement acceptable.

6. Composé selon la revendication 1 qui est le N-(2,4,5-triméthylphényl) (6-hydroxy-3,4-dihydro-2,5,7,8-tétraméthyl-2H[1]-benzopyran-2-yl) thiocarboxamide, ses isomères optiques, et ses sels d'addition à un acide ou à une base pharmaceutiquement acceptable.

7. Composé selon la revendication 1 qui est le N-(2,6-diméthylphényl) (6-hydroxy-3,4-dihydro-2,5,7,8-tétraméthyl-2H[1]-benzopyran-2-yl) thiocarboxamide, ses isomères optiques, et ses sels d'addition à un acide ou à une base pharmaceutiquement acceptable.

8. Composé selon la revendication 1 qui est le N-(3,4,5-triméthoxyphényl) (6-hydroxy-3,4-dihydro-2,5,7,8-tétraméthyl-2H[1]-benzopyran-2-yl) thiocarboxamide, ses isomères optiques, et ses sels d'addition à un acide ou à une base pharmaceutiquement acceptable.

9. Composé selon la revendication 1 qui est la 1-[(3,4-dihydro-6-hydroxy-2,5,7,8-tétraméthyl-2H-1-benzopyran-2-yl)thiocarbonyl]-4-(4-chlorobenzhydryl) pipérazine, ses isomères optiques et ses sels d'addition à un acide ou une base pharmaceutiquement acceptable.

10. Composé selon la revendication 1 qui est le N-(3,5-ditert-butyl-4-hydroxyphényl) (6-hydroxy-3,4-dihydro-2,5,7,8-tétraméthyl-2H[1]-benzopyran-2-yl)thiocarboxamide, ses isomères optiques et ses sels d'addition à une base pharmaceutiquement acceptable.

11. Composé selon la revendication 1 qui est la 1-[(3,4-dihydro-6-hydroxy-2,5,7,8-tétraméthyl-2H-1-benzopyran-2-yl)thiocarbonyl]-4-(4-fluorophényl)pipérazine, ses isomères optiques et ses sels d'addition à un acide ou à une base pharmaceutiquement acceptable.

12. Composé selon la revendication 1 qui est le N-phényl (3,4-dihydro-6-hydroxy-3,5,7,8-tétraméthyl-2H-1-benzopyran-2-yl)thiocarboxamide ses isomères optiques et ses sels d'addition à une base pharmaceutiquement acceptable.

13. Procédé d'obtention des composés de formule (I) selon la revendication 1, caractérisé en ce que l'on utilise comme matière première un composé de formule (II) : 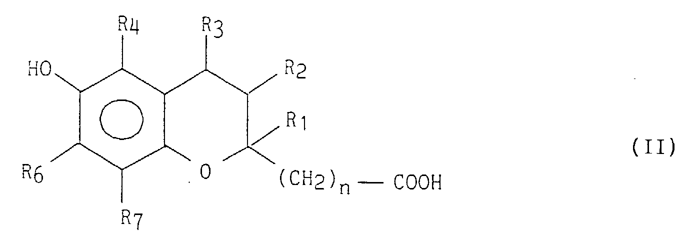 avec R₁, R₂, R₃, R₄, R₆, R₇ et n ayant la même définition que dans la revendication 1,
que l'on peut estérifier, en milieu basique anhydre, par un composé R₅''-Hal ou R₅''-O-R₅'', où Hal représente un atome d'halogène et où R₅'' représente un groupement acyle inférieur Rₐ-CO- dans lequel Ra est tel que défini dans la revendication 1,
pour obtenir un composé de formule (III) : 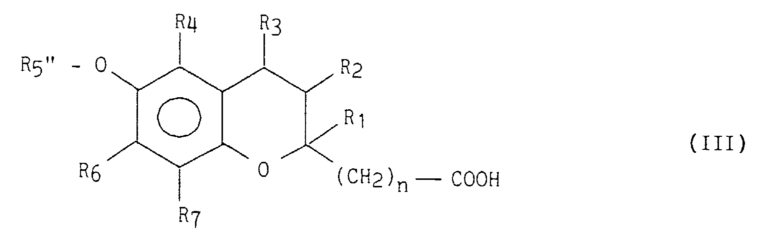 avec R₁, R₂, R₃, R₄, R₆, R₇, n, et R₅'' tels que définis précédemment, qui est transformé en son halogénure par action d'un agent d'halogénation, puis traité, dans un solvant approprié, en présence d'un agent alcalin, par une amine de formule (IV) : 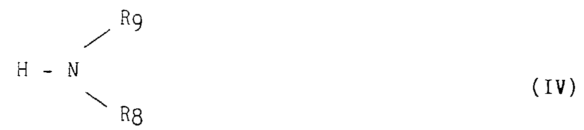 avec R₈ et R₉ ayant la même signification que dans la revendication 1 pour obtenir un composé de formule (Iₐ) : 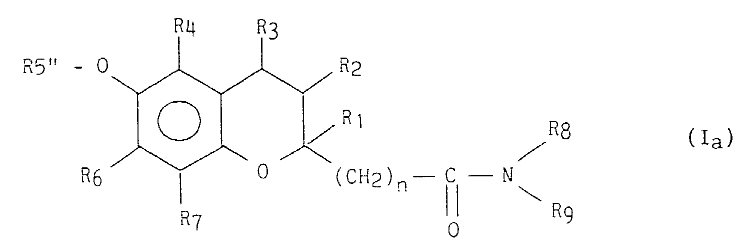 avec R₁, R₂, R₃, R₄, R₆, R₇, R₈, R₉, n, et R₅'' tels que définis précédemment,
que l'on peut ensuite,
- saponifier par action d'un hydroxyde de métal alcalin ou alcalino-terreux en un composé de formule (I_{b}) : 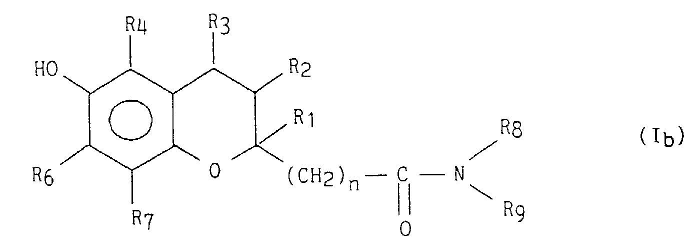 avec R₁, R₂, R₃, R₄, R₆, R₇, R₈, R₉ et n tels que définis précédemment,
- puis, le cas échéant, éthérifié par action d'un dérivé de formule R₅'''-O-R₅''' ou R₅'''-Hal', où Hal' représente un atome d'halogène et R₅''' représente un groupement alkyle inférieur Rₐ, un groupement acyle inférieur Ra-CO-, un groupement alcoxyalkyle de forme Rₐ-O-R_{b}-, un groupement alcoxycarbonyle de forme Ra-O-CO- un groupement alcoxycarbonylalkyle de forme Rₐ-O-CO-R_{b}-, ou un groupement carboxylalkyle de forme HOOC-Rₐ-, avec Rₐ et R_{b} tels que définis dans la revendication 1, pour obtenir un composé de formule (I_{c}) : 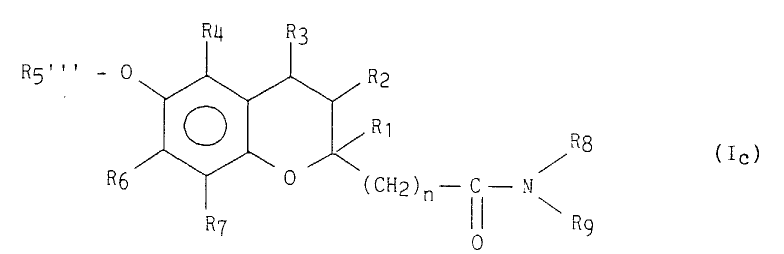 avec R₁, R₂, R₃, R₄, R₆, R₇, R₈, R₉, n, et R₅''' tels que définis précédemment,
composés de formule (Ia), (Ib), et (Ic) qui sont ensuite soumis à l'action du réactif de Lawesson pour obtenir les composés de formule (I) selon la revendication 1 correspondants,
ces composés de formule (I) pouvant être, si on le désire :
- purifiés,
- séparés en leurs isomères optiques, sous forme pure ou sous forme de mélange,
- ou transformés en leurs sels d'addition à une base ou à un acide pharmaceutiquement acceptable.

14. Procédé d'obtention des composés de formule (I') : 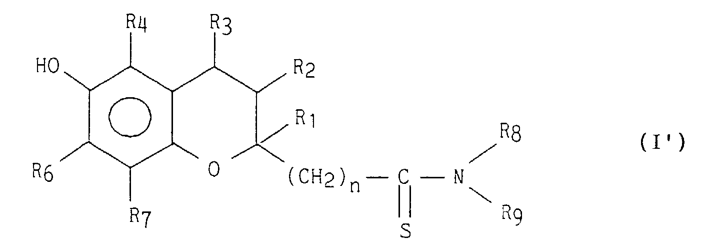 dans laquelle R₁, R₂, R₃, R₄, R₆, R₇, R₈, R₉ et n sont tels que définis dans la revendication 1,
cas particulier des composés de formule (I) selon la revendication 1 dans lesquels R₅ représente un atome d'hydrogène,
et des composés de formule (I'a) de formule : 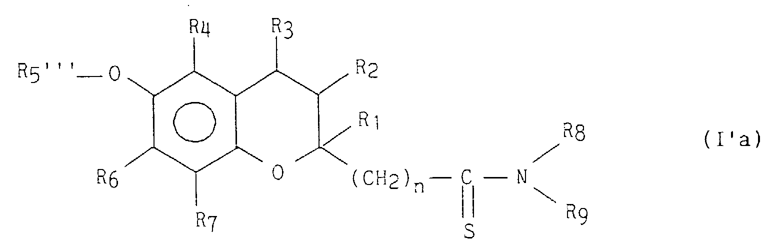 dans laquelle R₁, R₂, R₃, R₄, R₆, R₇, R₈, R₉, n sont tel que définis dans la formule (I) et R₅''' est tel que défini dans la revendication 13,
cas particulier des composés de formule (I) selon la revendication 1 dans lesquels R₅ représente un radical R₅''',
caractérisé en ce que l'on saponifie, par action d'un hydroxyde de métal alcalin ou alcalino-terreux, un composé de formule (I'') : 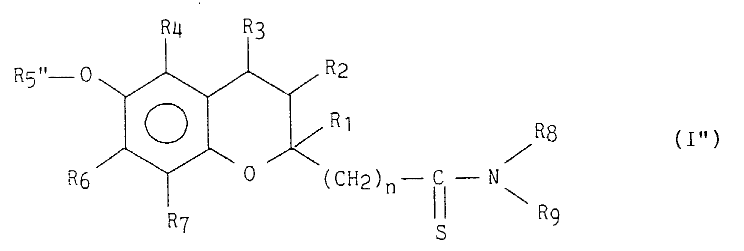 dans laquelle R₁, R₂, R₃, R₄, R₆, R₇, R₈, R₉, et n sont tels que définis précédemment et R₅'' représente un groupement acyle inférieur Ra-CO- dans lequel Ra est tel que défini dans la revendication 1, cas particulier des composés de formule (I) dans lesquels R₅ représente un radical R₅'', pour obtenir le composé de formule (I') correspondant, que l'on éthérifie ou estérifie, le cas échéant, par action d'un composé de formule R₅'''-O-R₅''' ou R₅'''-Hal'', où Hal'' représente un atome d'halogène et R₅''' est tel que défini précédemment, pour obtenir le composé de formule (I'a) correspondant,
composés de formule (I') et (I'a) qui peuvent être, si on le désire :
- purifiés,
- séparés en leurs isomères optiques, sous forme pure ou sous forme de mélange,
- ou transformés en leurs sels d'addition à une base ou à un acide pharmaceutiquement acceptable.

15. Procédé selon l'une des revendications 13 ou 14 dans lequel R₅'' représente un groupement acyle linéaire ou ramifié contenant 6, 7, ou 8 atomes de carbone.

16. Composition pharmaceutique contenant un composé selon la revendication 1 ou un de ses sels d'addition à un acide ou une base pharmaceutiquement acceptable, en combinaison avec un ou plusieurs excipients pharmacologiquement acceptables.

17. Composition pharmaceutique selon la revendication 16 contenant un composé de la présente invention peuvent ainsi être utilisés dans le traitement ou la prévention des désordres ischémiques centraux ou périphériques, des maladies inflammatoires, de l'arthrite rhumatoïde, des maladies métaboliques, de l'athérome, de l'artériosclérose, des maladies respiratoires, de l'asthme, de l'emphysème, des maladies d'origine immunologique, du lupus érythémateux, des réactions allergiques, du vieillissement cérébral ou cutané ainsi que dans la prévention et le traitement des dommages dus aux traumatismes chirurgicaux.

## Patentansprüche

1. Verbindungen der Formel (I): 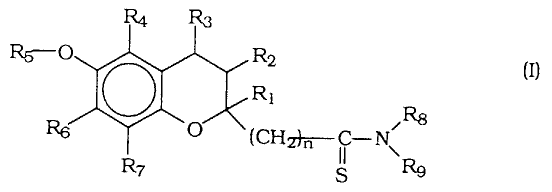 in der:
n eine ganze Zahl mit einem Wert von 0 oder 1.
R₁, R₂, R₃, R₄, R₆ und R₇, die gleichartig oder verschieden sein können, jeweils unabhängig voneinander ein Wasserstoffatom oder eine Niedrigalkylgruppe Rₐ-, worin Rₐ eine geradkettige oder verzweigte Alkylgruppe mit 1 bis 8 Kohlenstoffatomen darstellt,
R₅:
- ein Wasserstoffatom,
- eine Niedrigalkylgruppe Rₐ-,
- eine Niedrigacylgruppe Rₐ-CO-,
- eine Alkoxyalkylgruppe der Formel Rₐ-O-R_{b}-,
- eine Alkoxycarbonylgruppe der Formel Rₐ-O-CO-,
- eine Alkoxycarbonylalkylgruppe der Formel Rₐ-O-CO-R_{b}-,
- eine Carboxyalkylgruppe der Formel HOOC-Rₐ-,
worin Rₐ und R_{b}, die gleichartig oder verschieden sein können, jeweils unabhängig voneinander eine geradkettige oder verzweigte Alkylgruppe mit 1 bis 8 Kohlenstoffatomen darstellen,
R₈ und R₉
- entweder gemeinsam mit dem sie tragenden Stickstoffatom eine Gruppe ausgewählt aus:
· Piperazin.
· substituiertem Piperazin.
· Piperidin,
· substituiertem Piperidin,
· Pyrrolidin,
· substituiertem Pyrrolidin,
· Morpholin,
· durch eine oder mehrere Alkylgruppen substituiertem Morpholin,
· Tetrahydropyridin,
· Thiomorpholin,
· Azaspiran mit 5 bis 12 Kettengliedern,
· mit einer oder mehreren Alkylgruppen oder Oxogruppen substituiertem Aza-spiran mit 5 bis 12 Kettengliedern,
· mono- oder bicyclischem Azacycloalkyl mit 7 bis 12 Kettengliedern, welches gegebenenfalls in seinem Gerüst 1 bis 2 zusätzliche Heteroatome ausgewählt aus Sauerstoff, Schwefel und Stickstoff enthält,
· mit einer oder mehreren Alkylgruppen oder Oxogruppen substituiertem mono- oder bicyclischem Azacycloalkyl mit 7 bis 12 Kettengliedern, welches gegebenenfalls 1 bis 2 zusätzliche Heteroatome ausgewählt aus Sauestoff, Schwefel und Stickstoff enthält.
· einer Gruppe -NH-(CH₂)ₖ-NH₂, in der k eine ganze Zahl mit einem Wert von 2, 3 oder 4 darstellt, und
· einer substituierten Gruppe -NH-(CH₂)ₖ-NH₂, in der k die oben angegebenen Bedeutungen besitzt,
mit der Maßgabe, daß der Begriff "substituiert", wie er für die oben angesprochenen Piperazin-, Piperidin-, Pyrrolidingruppen und die Gruppe -NH-(CH₂)ₖ-NH₂ verwendet wird, bedeutet, daß diese Gruppen durch ein oder mehrere Halogenatome, Hydroxygruppen, Carboxylgruppen, Gruppen R₁₀ oder Gruppen 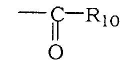 substituiert sind, worin R₁₀ ausgewählt ist aus:
· Alkyl,
· Alkoxy,
· Alkenyl,
· 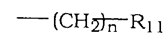 oder 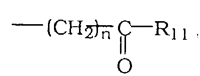 worin n 0 oder eine ganze Zahl mit einem Wert von 1 bis 5 und R₁₁ eine aus Phenyl, Benzhydryl, 1,1-Diphenylmethylidenyl, Thienyl, Pyrrolyl, Pyrrolidinyl, Furyl, Pyrimidinyl, Pyridyl, Benzodioxolyl, Benzodioxanyl, Naphthyl, Chinolinyl, Isochinolinyl, Cycloalkyl und Dicycloalkylmethyl, bedeuten, wobei der Begriff "Cycloalkyl" für eine mono- oder bicyclische Gruppe mit 3 bis 12 Kettengliedern steht, und diese Gruppen R₁₀ ihrerseits durch eine oder mehrere Gruppen ausgewählt aus Halogen, Trifluormethyl, Oxo, Carboxyl, Hydroxy, Alkyl, Alkoxy, Halogenalkoxy, Acetyl und Pyrrolidinyl substituiert sein können,
- oder R₈ und R₉, die gleichartig oder verschieden sind, jeweils unabhängig voneinander:
· ein Wasserstoffatom,
· eine Niedrigalkylgruppe Rₐ- oder eine substituierte Niedrigalkylgruppe Rₐ-,
· eine Niedrigalkenylgruppe oder eine substituierte Niedrigalkenylgruppe, wobei die Alkenylgruppe eine ungesättigte, geradkettige oder verzweigte Kohlenwasserstoffgruppe mit 2 bis 8 Kohlenstoffatomen darstellt,
· eine Gruppe A-(CH₂)ₘ- oder eine substituierte Gruppe A-(CH₂)ₘ-, in der m eine ganze Zahl mit einem Wert von 0, 1 oder 2 und A eine Cycloalkylgruppe mit p Kohlenstoffatomen, worin p für eine ganze Zahl mit einem Wert von 3 bis 7 steht, darstellen,
· eine Alkoxyalkylgruppe der Formel Rₐ-O-R_{b}- oder eine substituierte Alkoxyalkylgruppe der Formel Rₐ-O-R_{b}-, worin Rₐ und R_{b}, die gleichartig oder verschieden sein können, eine geradkettige oder verzweigte Niedrigalkylgruppe mit 1 bis 8 Kohlenstoffatomen darstellen,
· eine Alkoxycarbonylalkylgruppe der Formel Rₐ-O-CO-R_{b}- oder eine substituierte Alkoxycarbonylalkylgruppe der Formel Rₐ-O-CO-R_{b}-, worin Rₐ und R_{b} die oben angegebenen Bedeutungen besitzen,
· eine Gruppe B-(CH₂)_{q} oder eine substituierte Gruppe B-(CH₂)_{q}, in der q eine ganze Zahl mit einem Wert von 0, 1, 2 oder 3 und B eine Naphthalin-, 1,3-Dioxan-, Pyran- oder Benzopyrangruppe darstellen.
· eine Gruppe E-(CH₂)_{q}- oder eine substituierte Gruppe E-(CH₂)_{q}-, worin q die oben angegebenen Bedeutungen besitzt und E eine nichtsubstituierte oder substituierte Azaspiran- oder Azacycloalkylgruppe, wie sie oben definiert worden sind, darstellt,
· eine Pheny]-(CH₂)_{q}- gruppe oder eine substituierte Phenyl-(CH₂)_{q}-gruppe, worin q die oben angegebenen Bedeutungen besitzt,
· eine Heteroaryl-(CH₂)_{q}-gruppe oder eine substituierte Heteroaryl-(CH₂)_{q}-gruppe, worin q die oben angegebenen Bedeutungen besitzt, und Heteroaryl ausgewählt ist aus:
Furan, Chinolin, Isochinolin, Pyridin, Thiophen, Thiazol, Isothiazol, Oxazol, Isoxazol, Naphthyridin, Benzofuran, β-Carbolin oder γ-Carbolin,
· eine Guanidino- oder Amidinogruppe, die nichtsubstituiert oder durch eine oder mehrere geradkettige oder verzweigte Alkylgruppen mit 1 bis 6 Kohlenstoffatomen substituiert ist,
· einen der folgenden Reste D₁ bis D₄: 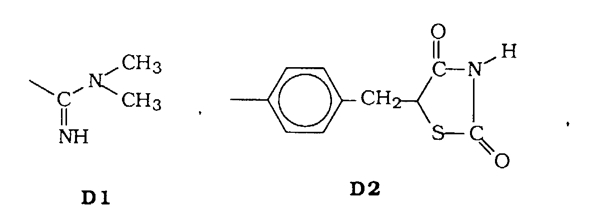 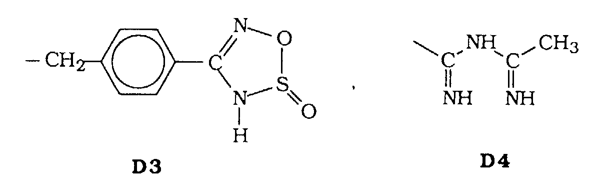 wobei es sich versteht, daß bei der Definition der allgemeinen Formel (I) der Begriff "substituiert", wie er für die oben definierten Gruppen benutzt worden ist, nämlich für Niedrigalkyl Rₐ-, Niedrigalkenyl, A-(CH₂)ₘ-, Alkoxyalkyl Rₐ-O-R_{b}-, Alkoxycarbonylalkyl Rₐ-O-CO-R_{b}-, B-(CH₂)_{q}-, Phenyl-(CH₂)_{q}-, Heteroaryl-(CH₂)_{q}- bedeutet, daß, wenn nichts Näheres angegeben ist, diese Gruppen durch einen oder mehrere gleichartige oder verschiedene Reste substituiert sein können, welche jeweils unabhängig voneinander:
eine Niedrigalkylgruppe R_{c}-,
eine Niedrigalkoxygruppe R_{c}-O-,
eine Niedrigacylgruppe R_{c}-CO-,
eine Trifluormethylgruppe,
eine Carboxylgruppe,
eine Hydroxygruppe,
eine Oxogruppe,
eine Guanidinogruppe,
eine Amidinogruppe
oder ein Halogenatom
darstellen, wobei R_{c} eine geradkettige oder verzweigte Alkylgruppe mit 1 bis 6 Kohlenstoffatomen darstellt, bedeuten.
deren optische Isomere in reiner Form oder in Form einer Mischung sowie gegebenenfalls deren Additionssalze mit einer pharmazeutisch annehmbaren Säure oder Base.

2. Verbindungen nach Anspruch 1, worin n O, R₈ ein Wasserstoffatom und R₉ eine Phenylgruppe oder eine Phenylgruppe, die durch eine oder mehrere Gruppen ausgewählt aus Niedrigalkyl R_{c}-, Niedrigalkoxy R_{c}-O-, Niedrigacyl R_{c}-CO-, Trifluormethyl, Carboxy, Hydroxy und Halogen substituiert ist, worin R_{c} eine geradkettige oder verzweigte Alkylgruppe mit 1 bis 6 Kohlenstoffatomen darstellt, bedeuten,
deren optische Isomere in reiner Form oder in Form einer Mischung sowie deren Additionssalze mit einer pharmazeutisch annehmbaren Säure oder Base.

3. Verbindungen nach Anspruch 1, worin R₅ ein Wasserstoffatom darstellt, deren optische Isomere in reiner Form oder in Form einer Mischung und deren Additionssalze mit einer pharmazeutisch annehmbaren Säure oder Base.

4. Verbindungen nach Anspruch 1, worin R₈ und R₉ gemeinsam mit dem sie tragenden Stickstoffatom ein nichtsubstituiertes oder substituiertes Piperazin bilden, deren optische Isomere und deren Additionssalze mit einer pharmazeutisch annehmbaren Säure oder Base.

5. Verbindung nach Anspruch 1, nämlich N-Phenyl-(6-hydroxy-3,4-dihydro-2,5,7,8-tetramethyl-2H[1]-benzopyran-2-yl)-thiocarboxamid, dessen optische Isomere und dessen Additionssalze mit einer pharmazeutisch annehmbaren Säure oder Base.

6. Verbindung nach Anspruch 1, nämlich N-(2,4,5-Trimethylphenyl)-(6-hydroxy-3,4-dihydro-2,5,7,8-tetramethyl-2H[1]-benzopyran-2-y])-thiocarboxamid, dessen optische Isomere und dessen Additionssalze mit einer pharmazeutisch annehmbaren Säure oder Base.

7. Verbindung nach Anspruch 1, nämlich N-(2,6-Dimethylphenyl)-(6-hydroxy-3,4-dihydro-2,5,7,8-tetramethyl-2H[1]-benzopyran-2-yl)-thiocarboxamid, dessen optische Isomere und dessen Additionssalze mit einer pharmazeutisch annehmbaren Säure oder Base.

8. Verbindung nach Anspruch 1, nämlich N-(3,4,5-Trimethoxypheny])-(6-hydroxy-3,4-dihydro-2,5,7,8-tetramethyl-2H[1]-benzopyran-2-yl)-thiocarboxamid, dessen optische Isomere und dessen Additionssalze mit einer pharmazeutisch annehmbaren Säure oder Base.

9. Verbindung nach Anspruch 1, nämlich 1-[(3,4-Dihydro-6-hydroxy-2,5,7,8-tetramethyl-2H[1]-benzopyran-2-yl)-thiocarbonyl]-4-(4-chlorbenzhydryl)-piperazin, dessen optische Isomere und dessen Additionssalze mit einer pharmazeutisch annehmbaren Säure oder Base.

10. Verbindung nach Anspruch 1, nämlich N-(3,5-Ditert.-butyl-4-hydroxyphenyl)-(6-hydroxy-3,4-dihydro-2,5,7,8-tetramethyl-2H[1]-benzopyran-2-yl)-thiocarboxamid, dessen optische Isomere und dessen Additionssalze mit einer pharmazeutisch annehmbaren Säure oder Base.

11. Verbindung nach Anspruch 1, nämlich 1-[(3.4-Dihydro-6-hydroxy-2,5,7,8-tetramethyl-2H-1-benzopyran-2-yl)-thiocarbonyll-4-(4-fluorphenyl)-piperazin, dessen optische Isomere und dessen Additionssalze mit einer pharmazeutisch annehmbaren Säure oder Base.

12. Verbindung nach Anspruch 1, nämlich N-Phenyl-(3,4-dihydro-6-hydroxy-3,5,7,8-tetramethyl-2H-1-benzopyran-2-yl)-thiocarboxamid, dessen optische Isomere und dessen Additionssalze mit einer pharmazeutisch annehmbaren Säure oder Base.

13. Verfahren zur Herstellung der Verbindungen der Formel (I) nach Anspruch 1, dadurch gekennzeichnet, daß man als Ausgangsmaterial eine Verbindung der Formel (II) verwendet: 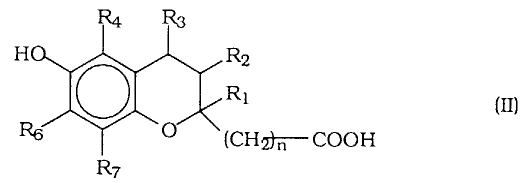 in der R₁, R₂, R₃, R₄, R₆, R₇ und n die in Anspruch 1 angegebenen Bedeutungen besitzen,
welche man in wasserfreiem basischem Medium mit einer Verbindung R₅"-Hal oder R₅"-O-R₅", in der Hal ein Halogenatom und R₅" eine Niedrigacylgruppe Rₐ-CO- bedeuten, worin Rₐ die in Anspruch 1 angegebenen Bedeutungen besitzt, verestern kann zur Bildung einer Verbindung der Formel (III): 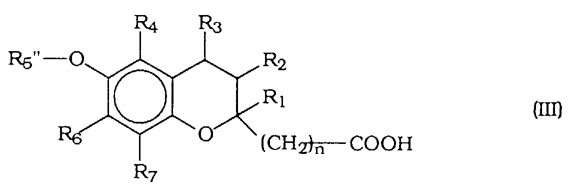 in der R₁, R₂, R₃, R₄, R₆, R₇, n und R₅" die oben angegebenen Bedeutungen besitzen, welche durch Einwirkung eines Halogenierungsmittels in ihr Halogenid umgewandelt und dann mit einem geeigneten Lösungsmittel in Gegenwart eines alkalischen Mittels mit einem Amin der Formel (IV): 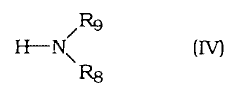 in der R₈ und R₉ die in Anspruch 1 angegebenen Bedeutungen besitzen, behandelt wird zur Bildung einer Verbindung der Formel (Iₐ): 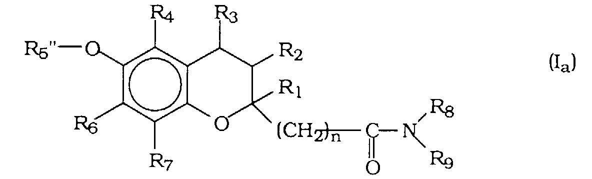 in der R₁, R₂, R₃, R₄, R₆, R₇, R₈, R₉, n und R₅" die oben angegebenen Bedeutungen besitzen.
welche man anschließend
- durch Einwirkung eines Alkalimetallhydroxids oder eines Erdalkalimetallhydroxids zu einer Verbindung der Formel (I_{b}) verseifen kann: 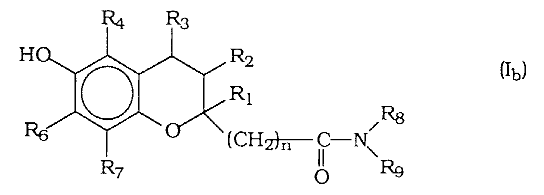 in der R₁, R₂, R₄, R₆, R₇, R₈, R₉ und n die oben angegebenen Bedeutungen besitzen,
- und dann gegebenenfalls durch Einwirkung eines Derivats der Formel R₅"'-O-R₅"' oder R₅"'-Hal' in der Hal' ein Halogenatom und R₅"' eine Niedrigalkylgruppe Rₐ, eine Niedrigacylgruppe Rₐ-CO-, eine Alkoxyalkylgruppe der Formel Rₐ-O-R_{b}-, eine Alkoxycarbonylgruppe der Formel Rₐ-O-CO-, eine Alkoxycarbonylalkylgruppe der Formel Rₐ-O-CO-R_{b}- oder eine Carboxyalkylgruppe der Formel HOOC-Rₐ-, worin Rₐ und R_{b} die in Anspruch 1 angegebenen Bedeutungen besitzen, darstellen, verethern kann zur Bildung einer Verbindung der Formel (I_{c}): 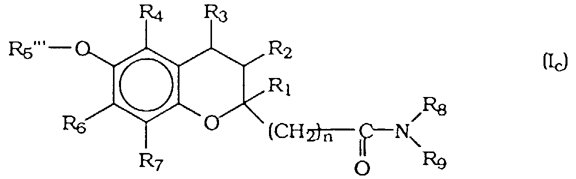 in der R₁, R₂, R₃, R₄, R₆, R₇, R₈, R₉, n und R₅"' die oben angegebenen Bedeutungen besitzen,
welche Verbindungen der Formeln (Iₐ), (I_{b}) und (I_{c}) anschließend der Einwirkung des Lawesson-Reagens unterworfen werden zur Bildung der entsprechenden Verbindungen der Formel (I) nach Anspruch 1,
welche Verbindungen der Formel (I) gewünschtenfalls:
- gereinigt werden können,
- in ihre optischen Isomeren in reiner Form oder in Form einer Mischung aufgetrennt werden können oder
- mit einer pharmazeutisch annehmbaren Base oder Säure in ihre Additionssalze umgewandelt werden können.

14. Verfahren zur Herstellung der Verbindungen der Formel (I'): 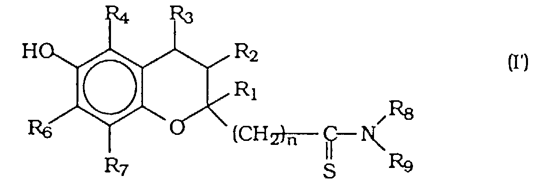 in der R₁, R₂, R₃, R₄, R₆, R₇, R₈, R₉ und n die in Anspruch 1 angegebenen Bedeutungen besitzen,
einem Sonderfall der Verbindungen der Formel (I) nach Anspruch 1, in der R₅ ein Wasserstoffatom darstellt,
und der Verbindungen der Formel (I'ₐ): 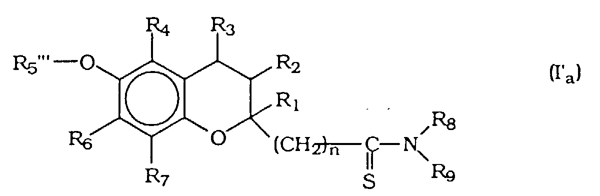 in der R_{1,} R₂, R₃, R₄, R₆, R₇, R₈, R₉ und n die bezüglich der Formel (I) angegebenen Bedeutungen besitzen und R₅'" die in Anspruch 13 angegebenen Bedeutungen besitzt,
einem Sonderfall der Verbindungen der Formel (I) nach Anspruch 1, worin R₅ eine Gruppe R₅"' darstellt,
dadurch gekennzeichnet, daß man eine Verbindung der Formel (I"): 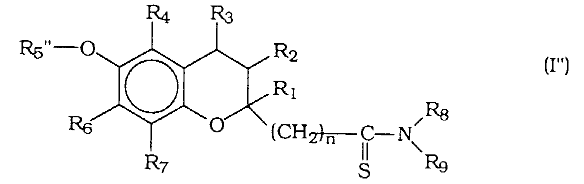 in der R₁, R₂, R₃, R₄, R₆, R₇, R₈, R₉ und n die oben angegebenen Bedeutungen besitzen und R₅" eine Niedrigacylgruppe Rₐ-CO- darstellt, in der Rₐ die in Anspruch 1 angegebenen Bedeutungen besitzt, einem Sonderfall der Verbindungen der Formel (I), in denen R₅ eine Gruppe R₅" darstellt, durch Einwirkung eines Alkalimetall- oder Erdalkalimetallhydroxids verseift zur Bildung der entsprechenden Verbindung der Formel (I'). welche man gegebenenfalls durch Einwirkung einer Verbindung der Formel R₅"'-O-R₅"' oder R₅"'-Hal", worin Hal" ein Halogenatom darstellt und R₅"' die oben angegebenen Bedeutungen besitzt, verethert oder verestert zur Bildung der entsprechenden Verbindung der Formel (I'ₐ), welche Verbindungen der Formeln (I') und (I'ₐ) gewünschtenfalls:
- gereinigt werden können,
- in ihre optischen Isomeren in reiner Form oder in Form einer Mischung getrennt werden können oder
- mit einer pharmazeutisch annehmbaren Base oder Säure in ihre Additionssalze umgewandelt werden können.

15. Verfahren nach einem der Ansprüche 13 oder 14, worin R₅" eine geradkettige oder verzweigte Acylgruppe mit 6, 7 oder 8 Kohlenstoffatomen darstellt.

16. Pharmazeutische Zubereitung enthaltend eine Verbindung nach Anspruch 1 oder eines ihrer Additionssalze mit einer pharmazeutisch annehmbaren Säure oder Base in Kombination mit einem oder mehreren pharmakologisch annehmbaren Trägermaterialien.

17. Pharmazeutische Zubereitung nach Anspruch 16, enthaltend eine Verbindung der vorliegenden Erfindung zur Verwendung bei der Behandlung oder der Vorbeugung von zentralen oder peripheren ischämischen Störungen, Entzündungserkrankungen, rheumatoider Arthritis, Stoffwechselerkrankungen, Atheromen, Arteriosklerose, Atmungserkrankungen, Asthma, Emphysemen, Erkrankungen mit immunologischem Ursprung, Lupus erythematodes, allergischen Reaktionen, des Alterns des Gehirns oder der Haut sowie bei der Vorbeugung und der Behandlung von Schädigungen im Rahmen von chirurgischen Eingriffen.

## Claims

1. Compounds of formula (I): 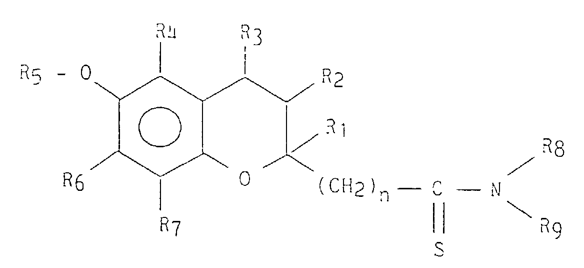 wherein :
n represents an integer 0 or 1,
R₁, R₂, R₃, R₄, R₆ and R₇, which may be identical or different, each represents independently of the other a hydrogen atom or a lower alkyl radical Rₐ- wherein Rₐ represents a linear or branched alkyl group having from 1 to 8 carbon atoms,
R₅ represents :
- a hydrogen atom,
- a lower alkyl group Rₐ-,
- a lower acyl group Rₐ-CO-,
- an alkoxyalkyl group of formula Rₐ-O-R_{b}-,
- an alkoxycarbonyl group of formula Rₐ-O-CO-,
- an alkoxycarbonylalkyl group of formula Rₐ-O-CO-R_{b}-,
- a carboxyalkyl group of formula HOOC-Rₐ-,
in which formulae Rₐ and R_{b}, which may be identical or different, each represents independently of the other a linear or branched alkyl radical having from 1 to 8 carbon atoms,
either
R₈ and R₉ form together with the nitrogen atom carrying them a group selected from :
. piperazine,
. substituted piperazine,
. piperidine,
. substituted piperidine,
. pyrrolidine,
. substituted pyrrolidine,
. morpholine,
. morpholine substituted by one or more alkyl radicals,
. tetrahydropyridine,
. thiomorpholine,
. azaspirane having from 5 to 12 ring members,
. azaspirane having from 5 to 12 ring members and substituted by one or more alkyl radicals or oxo groups,
. mono- or bi-cyclic azacycloalkyl having from 7 to 12 ring members and optionally including in its skeleton 1 or 2 additional hetero atoms selected from oxygen, sulphur and nitrogen,
. mono- or bi-cyclic azacycloalkyl having from 7 to 12 ring members and substituted by one or more alkyl radicals or oxo groups, and optionally including 1 or 2 additional hetero atoms selected from oxygen, sulphur and nitrogen,
. a -NH-(CH₂)ₖ-NH₂ group wherein k represents an integer 2, 3 or 4,
. and a substituted -NH-(CH₂)ₖ-NH₂ group wherein k is as defined above,
it being understood that the term "substituted" qualifying the groups piperazine, piperidine, pyrrolidine and -NH-(CH₂)ₖ-NH₂ above denotes that those groups may be substituted by one or more halogen atoms, hydroxy radicals, carboxy radicals, R₁₀ radicals or radicals 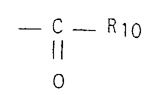 wherein R₁₀ is selected from :
. alkyl,
. alkoxy,
. alkenyl,
. -(CH₂)ₙ-R₁₁ and 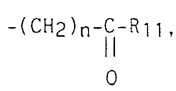 wherein n represents 0 or an integer from 1 to 5 and wherein R₁₁ represents a radical selected from phenyl, benzhydryl, 1,1-diphenylmethylidenyl, thienyl, pyrrolyl, pyrrolidinyl, furyl, pyrimidinyl, pyridyl, benzodioxolyl, benzodioxanyl, naphthyl, quinolinyl, isoquinolinyl, cycloalkyl and dicycloalkylmethyl; the term "cycloalkyl" representing a mono- or bi-cyclic group having from 3 to 12 ring members, it being possible for the R₁₀ radicals themselves to be substituted by one or more radicals selected from halogen, trifluoromethyl, oxo, carboxy, hydroxy, alkyl, alkoxy, haloalkoxy, acetyl and pyrrolidinyl,
or
- R₈ and R₉, which may be identical or different, each represents independently of the other:
. a hydrogen atom,
. a lower alkyl group Rₐ- or a substituted lower alkyl group Rₐ-,
. a lower alkenyl group or a substituted lower alkenyl group, wherein the alkenyl group is a linear or branched unsaturated hydrocarbon group having from 2 to 8 carbon atoms,
. a A-(CH₂)ₘ- group or a substituted A-(CH₂)ₘ- group, wherein m is an integer 0, 1 or 2 and A represents a cycloalkyl group having p carbon atoms, p being an integer from 3 to 7,
. an alkoxyalkyl group of formula Rₐ-O-R_{b}- or a substituted alkoxyalkyl group of formula Rₐ-O-R_{b}-, wherein Rₐ and R_{b}, which may be identical or different, each represents a linear or branched lower alkyl radical having from 1 to 8 carbon atoms,
. an alkoxycarbonylalkyl group of formula Rₐ-O-CO-R_{b}- or a substituted alkoxycarbonylalkyl group of formula Rₐ-O-CO-R_{b}-, wherein Rₐ and R_{b} are as defined above,
. a B-(CH₂)_{q}- group or a substituted B-(CH₂)_{q}- group, wherein q is an integer 0, 1, 2 or 3 and B represents a naphthalene, 1,3-dioxane, pyran or benzopyran radical,
. an E-(CH₂)_{q}- group or a substituted E-(CH₂)_{q}- group, wherein q is as defined above and E represents an unsubstituted or substituted azaspirane or azacycloalkyl group as defined above,
. a phenyl-(CH₂)_{q}- group or a substituted phenyl-(CH₂)_{q}- group, wherein q is as defined above,
. a heteroaryl-(CH₂)_{q}- group or a substituted heteroaryl-(CH₂)_{q}- group, wherein q is as defined above and wherein heteroaryl is selected from :
furan, quinoline, isoquinoline, pyridine, thiophene, thiazole, isothiazole, oxazole, isoxazole, naphthyridine, benzofuran, β-carboline or γ-carboline,
. a guanidino or amidino group, unsubstituted or substituted by one or more linear or branched alkyl radicals having from 1 to 6 carbon atoms,
. one of the following radicals D₁ to D₄ : 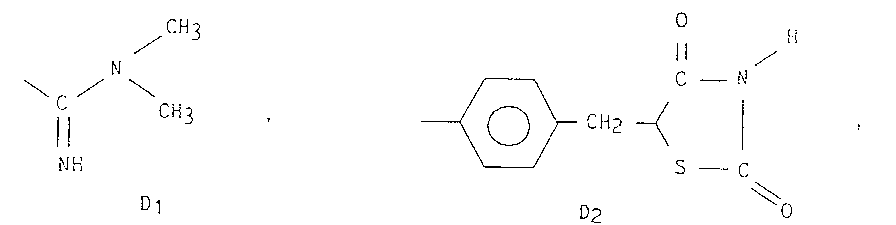 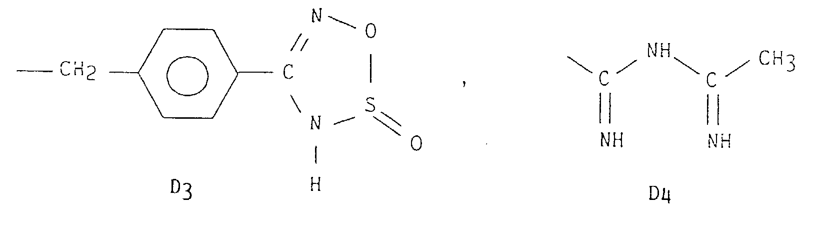 it being understood
in this description of the general formula (I) that the term "substituted" qualifying the groups as defined above : lower alkyl Rₐ-, lower alkenyl, A-(CH₂)ₘ-, alkoxyalkyl Rₐ-O-R_{b}-, alkoxycarbonylalkyl Rₐ-O-CO-R_{b}-, B-(CH₂)_{q}-, phenyl-(CH₂)_{q}-, heteroaryl-(CH₂)_{q}-, denotes, unless defined otherwise, that those groups may be substituted by one or more identical or different radicals each representing independently of the other(s):
lower alkyl R_{c}-
lower alkoxy R_{c}-O-,
lower acyl R_{c}-CO-,
trifluoromethyl,
carboxy,
hydroxy,
oxo,
guanidino,
amidino,
or a halogen atom
wherein R_{c} represents a linear or branched alkyl group having from 1 to 6 carbon atoms,
their optical isomers, in pure form or in the form of a mixture, and also, optionally, the addition salts thereof with a pharmaceutically acceptable acid or base.

2. Compounds according to claim 1, wherein n represents 0, R₈ represents a hydrogen atom and R₉ represents a phenyl group, or a phenyl group substituted by one or more radicals selected from lower alkyl R_{c}-, lower alkoxy R_{c}-O-, lower acyl R_{c}-CO-, trifluoromethyl, carboxy, hydroxy and halogen, R_{c} denoting a linear or branched alkyl group having from 1 to 6 carbon atoms,
their optical isomers, in pure form or in the form of a mixture,
and also the addition salts thereof with a pharmaceutically acceptable acid or base.

3. Compounds according to claim 1, wherein R₅ represents a hydrogen atom, their optical isomers, in pure form or in the form of a mixture,
and the addition salts thereof with a pharmaceutically acceptable acid or base.

4. Compounds according to claim 1, wherein R₈ and R₉ form together with the nitrogen atom carrying them an unsubstituted or substituted piperazine,
their optical isomers,
and the addition salts thereof with a pharmaceutically acceptable acid or base.

5. Compound according to claim 1, which is N-phenyl-(6-hydroxy-3,4-dihydro-2,5,7,8-tetramethyl-2H[1]-benzopyran-2-yl)thiocarboxamide, its optical isomers, and its addition salts with a pharmaceutically acceptable acid or base.

6. Compound according to claim 1, which is N-(2,4,5-trimethylphenyl)-(6-hydroxy-3,4-dihydro-2,5,7,8-tetramethyl-2H[1]-benzopyran-2-yl)thiocarboxamide, its optical isomers, and its addition salts with a pharmaceutically acceptable acid or base.

7. Compound according to claim 1, which is N-(2,6-dimethylphenyl)-(6-hydroxy-3,4-dihydro-2,5,7,8-tetramethyl-2H[1]-benzopyran-2-yl)thiocarboxamide, its optical isomers, and its addition salts with a pharmaceutically acceptable acid or base.

8. Compound according to claim 1, which is N-(3,4,5-trimethoxyphenyl)-(6-hydroxy-3,4-dihydro-2,5,7,8-tetramethyl-2H[1]-benzopyran-2-yl)thiocarboxamide, its optical isomers, and its addition salts with a pharmaceutically acceptable acid or base.

9. Compound according to claim 1, which is 1-[(3,4-dihydro-6-hydroxy-2,5,7,8-tetramethyl-2H[1]-benzopyran-2-yl)thiocarbonyl]-4-(4-chlorobenzhydryl)piperazine, its optical isomers, and its addition salts with a pharmaceutically acceptable acid or base.

10. Compound according to claim 1, which is N-(3,5-di-tert-butyl-4-hydroxyphenyl)-(6-hydroxy-3,4-dihydro-2,5,7,8-tetramethyl-2H[1]-benzopyran-2-yl)thiocarboxamide, its optical isomers, and its addition salts with a pharmaceutically acceptable acid or base.

11. Compound according to claim 1, which is 1-[(3,4-dihydro-6-hydroxy-2,5,7,8-tetramethyl-2H[1]-benzopyran-2-yl)thiocarbonyl]-4-(4-fluorophenyl)piperazine, its optical isomers, and its addition salts with a pharmaceutically acceptable acid or base.

12. Compound according to claim 1, which is N-phenyl-(3,4-dihydro-6-hydroxy-3,5,7,8-tetramethyl-2H[1]-benzopyran-2-yl)thiocarboxamide, its optical isomers, and its addition salts with a pharmaceutically acceptable acid or base.

13. Process for obtaining compounds of formula (I) according to claim 1, characterised in that there is used as starting material a compound of formula (II) : 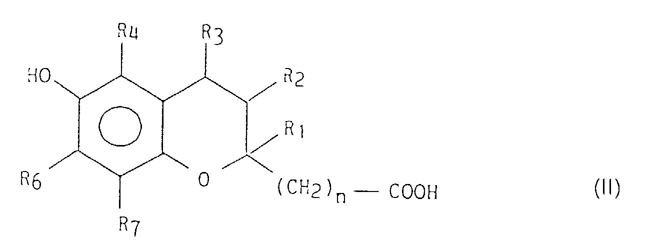 wherein R₁, R₂, R₃, R₄, R₆, R₇ and n are as defined in claim 1,
which may be esterified, in anhydrous basic medium, with a compound R₅"-Hal or R₅"-O-R₅" wherein Hal represents a halogen atom and R₅" represents a lower acyl group Rₐ-CO- in which Rₐ is as defined in claim 1,
to obtain a compound of formula (III) : 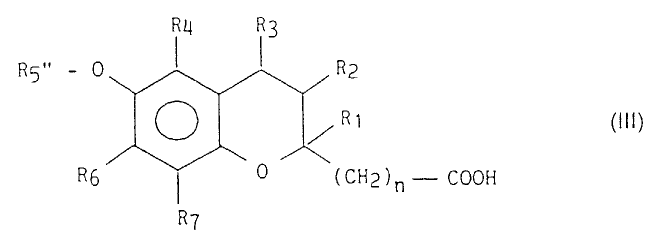 wherein R₁, R₂, R₃, R₄, R₆, R₇, n and R₅" are as defined above, which is converted into its halide by the action of a halogenation agent, then treated, in an appropriate solvent, in the presence of an alkaline agent, with a amine of formula (IV): 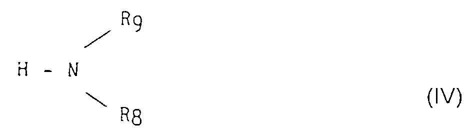 wherein R₈ and R₉ are as defined in claim 1, to obtain a compound of formula (Iₐ) : 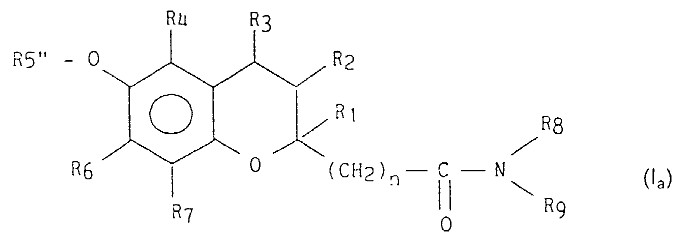 wherein R₁, R₂, R₃, R₄, R₆, R₇, R₆, R₉, n and R₅" are as defined above,
which may then be
- hydrolysed by the action of an alkali metal or alkaline earth metal hydroxide to form a compound of formula (I_{b}) : 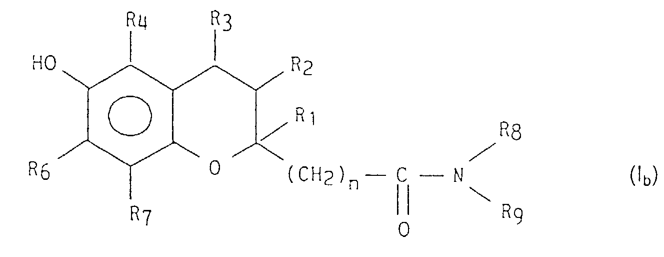 wherein R₁, R₂, R₃, R₄, R₆, R₇, R₈, R₉ and n are as defined above,
- then, as the case may be, etherified by the action of a compound of formula R₅"'-O-R₅"' or R₅"'-Hal' wherein Hal' represents a halogen atom and R₅"' represents a lower alkyl group Rₐ, a lower acyl group Rₐ-CO-, an alkoxyalkyl group of formula Rₐ-O-R_{b}-, an alkoxycarbonyl group of formula Rₐ-O-CO-, an alkoxycarbonylalkyl group of formula Rₐ-O-CO-R_{b}- or a carboxyalkyl group of formula HOOC-Rₐ-, wherein Rₐ and R_{b} are as defined in claim 1, to obtain a compound of formula (I_{c}) : 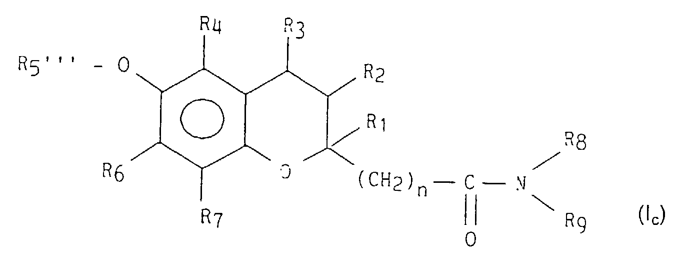 wherein R₁, R₂, R₃, R₄, R₆, R₇, R₈, R₉, n and R₅"' are as defined above,
which compounds of formula (Iₐ), (I_{b}) and (I_{c}) are than subjected to the action of Lawesson's reagent to obtain the corresponding compounds of formula (I) according to claim 1,
it being possible, if desired, for those compounds of formula (I) to be :
- purified,
- separated into their optical isomers, in pure form or in the form of a mixture,
- or converted into their addition salts with a pharmaceutically acceptable acid or base.

14. Process for obtaining compounds of formula (I') : 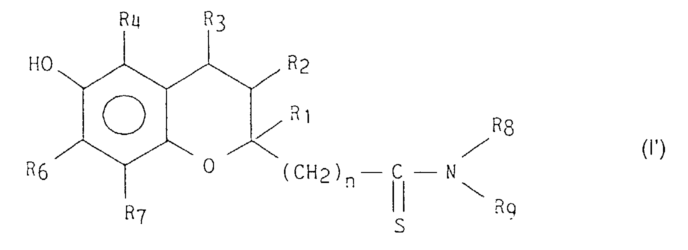 wherein R₁, R₂, R₃, R₄, R₆, R₇, R₈, R₉ and n are as defined in claim 1, a particular case of compounds of formula (I) according to claim 1 wherein R₅ represents a hydrogen atom,
and compounds of formula (I'a) of formula : 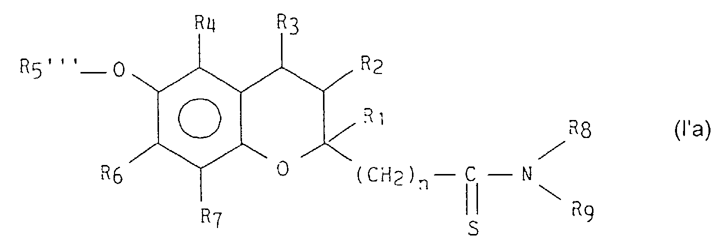 wherein R₁, R₂, R₃, R₄, R₆, R₇, R₈, R₉ and n are as defined for formula (I) and R₅"' is as defined in claim 13,
a particular case of compounds of formula (I) according to claim 1 wherein R₅ represents a radical R₅"',
characterised in that a compound of formula (I") : 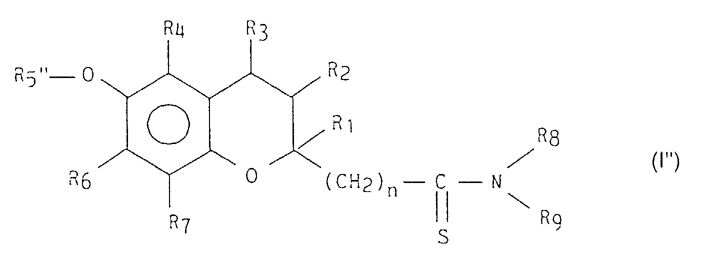 wherein R₁, R₂, R₃, R₄, R₆, R₇, R₈, R₉ and n are as defined above and R₅" represents a lower acyl group Rₐ-CO- wherein Rₐ is as defined in claim 1, a particular case of compounds of formula (I) wherein R₅ represents a radical R₅", is hydrolysed by the action of an alkali metal or alkaline earth metal hydroxide to obtain the corresponding compound of formula (I'), which is etherified or esterified, as the case may be, by the action of a compound of the formula R₅"'-O-R₅"' or R₅"'-Hal", wherein Hal" represents a halogen atom and R₅"' is as defined above, to obtain the corresponding compound of formula (I'a),
which compounds of formula (I') and (I'a) may, if desired, be :
- purified,
- separated into their optical isomers, in pure form or in the form of a mixture,
- or converted into their addition salts with a pharmaceutically acceptable acid or base.

15. Process according to either claim 13 or 14 wherein R₅" represents a linear or branched acyl group containing 6, 7 or 8 carbon atoms.

16. Pharmaceutical composition comprising a compound according to claim 1, or an addition salt thereof with a pharmaceutically acceptable acid or base, in combination with one or more pharmacologically acceptable excipients.

17. Pharmaceutical composition according to claim 16 comprising a compound of the present invention for use in the treatment or prevention of central or peripheral ischaemic disorders, inflammatory disorders, rheumatoid arthritis, metabolic disorders, atheroma, arteriosclerosis, respiratory disorders, asthma, emphysema, diseases of immunological origin, lupus erythematosus, allergic reactions, cerebral or cutaneous ageing and also in the prevention and treatment of damage resulting from surgical trauma.
